(19) Europäisches Patentamt
European Patent Office
Office européen des brevets

(11) **EP 4 250 425 A1**

(12) **EUROPEAN PATENT APPLICATION**
published in accordance with Art. 153(4) EPC

(43) Date of publication:
**27.09.2023 Bulletin 2023/39**

(21) Application number: **21905723.9**

(22) Date of filing: **15.12.2021**

(51) International Patent Classification (IPC):
***H01M 10/0569*** *(2010.01)* ***H01M 10/0567*** *(2010.01)*

(52) Cooperative Patent Classification (CPC):
**H01M 10/0525; H01M 10/0567; H01M 10/0568; H01M 10/0569**

(86) International application number:
**PCT/CN2021/138148**

(87) International publication number:
**WO 2022/127796 (23.06.2022 Gazette 2022/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.12.2020 CN 202011490108**

(71) Applicants:
- **Huawei Technologies Co., Ltd.**
  **Shenzhen, Guangdong 518129 (CN)**
- **Shenzhen Capchem Technology Co., Ltd.**
  **Shenzhen, Guangdong 518118 (CN)**

(72) Inventors:
- **MA, Qiang**
  **Shenzhen, Guangdong 518129 (CN)**
- **DENG, Yonghong**
  **Shenzhen, Guangdong 518118 (CN)**
- **ZHANG, Guangzhao**
  **Shenzhen, Guangdong 518118 (CN)**
- **HONG, Xiang**
  **Shenzhen, Guangdong 518129 (CN)**

(74) Representative: **Thun, Clemens**
**Mitscherlich PartmbB**
**Patent- und Rechtsanwälte**
**Karlstraße 7**
**80333 München (DE)**

# (54) BATTERY ELECTROLYTE SOLUTION, SECONDARY BATTERY, AND TERMINAL

(57) This application provides a battery electrolytic solution, including an electrolyte salt and a non-aqueous organic solvent. The non-aqueous organic solvent includes a first organic solvent shown in a formula (I) and/or a second organic solvent shown in a formula (II): $R_1$-S(=O)$_x$-N(-$R_3$)-$R_2$ formula (I); and $R_1$-S(=O)$_x$-N(-$R_3$)-S(=O)$_y$-$R_4$ formula (II). $R_1$ and $R_4$ are separately selected from fluoroalkyl, fluoroalkoxy, fluoroalkenyl, fluoroalkenyloxy, fluoroaryl, or fluoroaryloxy. $R_2$ and $R_3$ are separately selected from alkyl, alkoxy, alkenyl, alkenyloxy, aryl, or aryloxy. x is 1 or 2, and y is 1 or 2. A total content in mass of the first organic solvent and/or the second organic solvent in the electrolytic solution ranges from 10% to 90%. The electrolytic solution includes the first organic solvent and/or the second organic solvent of a high content in mass, so that side reactions between the electrolytic solution and the negative electrode can be reduced, thereby improving coulombic efficiency and cycling performance of the battery.

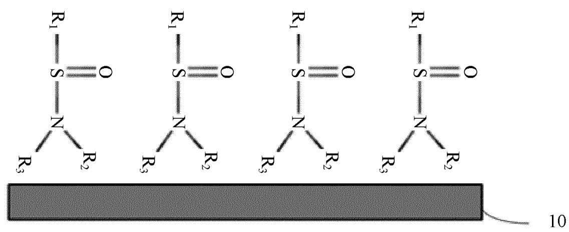

FIG. 2

EP 4 250 425 A1

## Description

This application claims priority to Chinese Patent Application No. 202011490108.5, filed with the China National Intellectual Property Administration on December 16, 2020 and entitled "BATTERY ELECTROLYTIC SOLUTION, SECONDARY BATTERY, AND TERMINAL", which is incorporated herein by reference in its entirety.

## TECHNICAL FIELD

Embodiments of this application relate to the field of secondary battery technologies, and in particular, to a battery electrolytic solution, a secondary battery, and a terminal.

## BACKGROUND

With the development of economies and technologies, various products, such as a portable electronic device (such as a smartphone, a digital camera, or a notebook computer), an uncrewed aerial vehicle, and an electric vehicle, urgently require an energy storage device with high energy density. Energy density of a commercial lithium-ion battery using graphite as a negative electrode material has been close to an upper limit, but still cannot satisfy endurance and standby needs of a user for the foregoing device. An effective way to improve energy density of a battery is to partially or entirely replace a graphite negative electrode with a negative electrode material with a higher theoretical capacity, such as a silicon-base material, a tin-base material, or a metal. However, due to large volume expansion and high activity, such type of negative electrode material with a higher theoretical capacity consumes a large amount of electrolytic solution during charging and discharging of the battery, resulting in low coulombic efficiency and poor cycling performance of the battery. Consequently, overall performance of the battery is poor.

Some functional additives are usually added into an electrolytic solution, to improve battery performance. However, existing functional additives have no significant effect on a negative electrode material with large volume expansion, such as a silicon-base material, a tin-base material, or a metal. In addition, the functional additives are generally added in a small amount (≤ 5 wt%), and are mostly exhausted through battery formation and capacity grading, resulting in poor cycling performance of the battery. This severely limits actual application of the negative electrode material in the battery.

## SUMMARY

In view of this, embodiments of this application provide a battery electrolytic solution, including a high content in mass of organic solvent with a special structure. The organic solvent with a special structure not only can isolate other solvents in the electrolytic solution from electrodes to avoid direct contact, but also can form a stable interface film on a negative electrode, thereby reducing side reactions between the electrolytic solution and the negative electrode, and improving coulombic efficiency and cycling performance of the battery.

Specifically, according to a first aspect, an embodiment of this application provides a battery electrolytic solution. The electrolytic solution includes an electrolyte salt and a non-aqueous organic solvent. The non-aqueous organic solvent includes a first organic solvent shown in a general structural formula (I) and/or a second organic solvent shown in a general structural formula (II):

$$R_1\text{-}S(=O)_x\text{-}N(\text{-}R_3)\text{-}R_2 \qquad \text{formula (I);}$$

and

$$R_1\text{-}S(=O)_x\text{-}N(\text{-}R_3)\text{-}S(=O)y\text{-}R_4 \qquad \text{formula (II).}$$

$R_1$ and $R_4$ are separately selected from one of fluoroalkyl, fluoroalkoxy, fluoroalkenyl, fluoroalkenyloxy, fluoroaryl, and fluoroaryloxy. $R_2$ and $R_3$ are separately selected from one of alkyl, alkoxy, alkenyl, alkenyloxy, aryl, and aryloxy. x is 1 or 2, and y is 1 or 2.

A total content in mass of the first organic solvent and/or the second organic solvent in the electrolytic solution ranges from 10% to 90%.

The battery electrolytic solution provided in the first aspect of embodiments of this application includes a high content in mass of first organic solvent and/or second organic solvent. The first organic solvent and the second organic solvent are similar to a surfactant and have a polar end and a non-polar or low-polar end that are opposite to each other. The non-polar or low-polar end is far away from an electrode side, which can effectively isolate other solvents in the electrolytic solution from the electrode to avoid direct contact, reduce side reactions, and improve coulombic efficiency

and cycling performance of the battery. Sulfinamide and/or sulfonamide on the polar end can be complexed with metal ions (for example, lithium ions) and more easily close to the electrode side, so that the metal ions (for example, lithium ions) easily reach a surface of the electrode, thereby reducing a probability of dendrite formation, reducing a short circuit risk, and improving battery safety performance. In addition, the first organic solvent and the second organic solvent are a substituted sulfinamide or substituted sulfonamide compound. The substituted sulfinamide or substituted sulfonamide compound can be preferentially reduced on the surface of the negative electrode than other components in the electrolytic solution, so that a stable interface film containing a metal fluoride, a metal nitride, a sulfide, and other compounds is formed on the surface of the negative electrode, reducing side reactions between the electrolytic solution and the negative electrode material, thereby improving coulombic efficiency and cycling stability of the battery.

In an implementation of this application, a number of carbon atoms in each of the fluoroalkyl, fluoroalkoxy, alkyl, and alkoxy ranges from 1 to 20; a number of carbon atoms in each of the fluoroalkenyl, fluoroalkenyloxy, alkenyl, and alkenyloxy ranges from 2 to 10; and a number of carbon atoms in each of the fluoroaryl, fluoroaryloxy, aryl, and aryloxy ranges from 6 to 20.

In this implementation of this application, carbon chains of $R_1$ and $R_4$ are relatively long, which helps electrolyte-phobicity to better isolate other solvents in the electrolytic solution from the electrode to avoid direct contact. However, an excessively long carbon chain causes poor compatibility with the electrolytic solution. In some implementations, a number of carbon atoms in each of $R_1$ and $R_4$ may range from 2 to 6.

In this implementation of this application, carbon chains of $R_2$ and $R_3$ are relatively short, which can reduce steric hindrance, and helps metal ions to be close to O and N in molecular structures of the first organic solvent and the second organic solvent, for better complexing the metal ions. In some implementations, a number of carbon atoms in each of $R_2$ and $R_3$ may range from 1 to 3.

In an implementation of this application, a total content in mass of the first organic solvent and/or the second organic solvent in the electrolytic solution ranges from 20% to 50%. An appropriate content can not only effectively reduce side reactions, but also not cause an excessive decrease of conductivity to reduce rate performance.

In an implementation of this application, in the battery electrolytic solution, a mass ratio of the second organic solvent to the first organic solvent ranges from 1: 1 to 3:1.

In an implementation of this application, the non-aqueous organic solvent further includes a co-solvent, and the co-solvent includes one or more of a carbonate ester-based solvent, a carboxylate ester-based solvent, and an ether-based solvent. The addition of the co-solvent can combine performance advantages of various solvents to improve comprehensive performance of the electrolytic solution.

In an implementation of this application, in the electrolytic solution, a ratio of a mass of the co-solvent to a total mass of the first organic solvent and/or the second organic solvent ranges from 1:80 to 8: 1.

In an implementation of this application, the electrolytic solution further includes an additive, and the additive includes one or more of biphenyl, fluorobenzene, vinylene carbonate, trifluoromethyl ethylene carbonate, vinyl ethylene carbonate, 1,3-propane sultone, 1,4-butane sultone, ethylene sulfate, ethylene sulfite, methylene methanedisulfonate, butanedinitrile, hexanedinitrile, 1,2-bis(2-cyanoethoxy)ethane, and 1,3,6-hexanetricarbonitrile. The addition of the additive can adapt to different application requirements to improve corresponding performance of the electrolytic solution.

In an implementation of this application, the electrolyte salt includes at least one of a lithium salt, a sodium salt, a potassium salt, a magnesium salt, a zinc salt, and an aluminum salt. The first organic solvent and the second organic solvent in embodiments of this application are applicable to various electrolyte systems.

In an implementation of this application, the electrolyte salt includes one or more of $MClO_4$, $MBF_4$, $MPF_6$, $MAsF_6$, $MPF_2O_2$, $MCF_3SO_3$, MTDI, $MB(C_2O_4)_2$, $MBF_2C_2O_4$, $M[(CF_3SO_2)_2N]$, $M[(FSO_2)_2N]$, and $M[(C_mF_{2m}+_1SO_2)(C_nF_{2n+1}SO_2) N]$, where M is Li, Na, or K, and m and n are natural numbers.

In an implementation of this application, a molar concentration of the electrolyte salt in the electrolytic solution ranges from 0.1 mol/L to 8.0 mol/L.

According to a second aspect, an embodiment of this application provides a secondary battery, including a positive electrode, a negative electrode, and a separator and an electrolytic solution between the positive electrode and the negative electrode. The electrolytic solution includes the battery electrolytic solution according to the first aspect in embodiments of this application.

In an implementation of this application, the negative electrode includes one or more of a carbon-based negative electrode, a silicon-based negative electrode, a tin-based negative electrode, a lithium negative electrode, a sodium negative electrode, a potassium negative electrode, a magnesium negative electrode, a zinc negative electrode, and an aluminum negative electrode.

In an implementation of this application, the carbon-based negative electrode includes one or more of graphite, hard carbon, soft carbon, and graphene; the silicon-based negative electrode includes one or more of silicon, silicon carbon, silicon oxygen, and a silicon metal compound; the tin-based negative electrode includes one or more of tin, tin carbon, tin oxygen, and a tin metal compound; and the lithium negative electrode includes a lithium metal or a lithium alloy.

In an implementation of this application, the lithium alloy includes at least one of a lithium silicon alloy, a lithium

sodium alloy, a lithium potassium alloy, a lithium aluminum alloy, a lithium tin alloy, and a lithium indium alloy.

In an implementation of this application, the secondary battery includes a lithium secondary battery, a potassium secondary battery, a sodium secondary battery, a magnesium secondary battery, a zinc secondary battery, or an aluminum secondary battery.

An embodiment of this application further provides a terminal, including a housing, and an electronic component and a battery that are accommodated in the housing. The battery supplies power to the electronic component. The battery includes the secondary battery according to the second aspect in embodiments of this application.

## BRIEF DESCRIPTION OF DRAWINGS

FIG. 1 is a schematic diagram of a structure of a secondary battery according to an embodiment of this application;
FIG. 2 is a schematic diagram of effect between a first organic solvent and an electrode according to an embodiment of this application;
FIG. 3 is a schematic diagram of a structure of a terminal according to an embodiment of this application;
FIG. 4 is a cycling curve graph of batteries in Examples 1 and 2 and Comparative Example 1 according to this application;
FIG. 5 is a cycling curve graph of batteries in Examples 3 and 4 and Comparative Example 2 according to this application;
FIG. 6 is a cycling curve graph of batteries in Examples 5 to 7 and Comparative Examples 3 to 5 according to this application;
FIG. 7 is a cycling curve graph of batteries in Examples 8 to 10 and Comparative Examples 6 and 7 according to this application; and
FIG. 8 is a cycling curve graph of batteries in Examples 11 and 12, Examples 16 and 17, and Comparative Examples 8 and 9 according to this application.

## DESCRIPTION OF EMBODIMENTS

The following describes embodiments of this application with reference to accompanying drawings in embodiments of this application.

As shown in FIG. 1, core components of a secondary battery (for example, a lithium-ion battery) include a positive electrode 101, a negative electrode 102, an electrolytic solution 103, a separator 104, a corresponding connecting accessory, and a loop. During charging, lithium ions deintercalate from lattices of a positive electrode material of the positive electrode 101, and is deposited to the negative electrode 102 after passing through the electrolytic solution 103. During discharging, lithium ions deintercalate from the negative electrode 102, and intercalate into the lattices of the positive electrode material after passing through the electrolytic solution 103. During charging and discharging, the electrolytic solution and the electrode materials react on a solid-liquid interface to form an interface film. The interface film significantly affects battery performance. An unstable interface film causes serious side reactions, reducing coulombic efficiency and battery cycling performance. To improve coulombic efficiency and cycling performance of a battery, embodiments of this application provide a battery electrolytic solution. The battery electrolytic solution includes a high content in mass of substituted sulfinamide or substituted sulfonamide compound. The addition of such compound can significantly improve performance of the electrolytic solution and improve comprehensive performance of the battery.

The battery electrolytic solution provided in embodiments of this application includes an electrolyte salt and a non-aqueous organic solvent. The non-aqueous organic solvent includes a first organic solvent shown in a general structural formula (I) and/or a second organic solvent shown in a general structural formula (II):

$$R_1\text{-}S(=O)_x\text{-}N(\text{-}R_3)\text{-}R_2 \qquad \text{formula (I);}$$

and

$$R_1\text{-}S(=O)_x\text{-}N(\text{-}R_3)\text{-}S(=O)_y\text{-}R_4 \qquad \text{formula (II).}$$

$R_1$ and $R_4$ are separately selected from one of fluoroalkyl, fluoroalkoxy, fluoroalkenyl, fluoroalkenyloxy, fluoroaryl, and fluoroaryloxy. $R_2$ and $R_3$ are separately selected from one of alkyl, alkoxy, alkenyl, alkenyloxy, aryl, and aryloxy. x is 1 or 2, and y is 1 or 2. A total content in mass of the first organic solvent and/or the second organic solvent in the electrolytic solution ranges from 10% to 90%.

According to the battery electrolytic solution provided in embodiments of this application, the organic solvent

includes the first organic solvent and/or the second organic solvent. The first organic solvent and the second organic solvent are a substituted sulfinamide or substituted sulfonamide compound. Some substituents are polar functional groups, and other substituents are non-polar or low-polar functional groups. Therefore, the first organic solvent and the second organic solvent are similar to a surfactant and have a polar end and a non-polar or low-polar end that are opposite to each other. Refer to FIG. 2. The special structure has the following effects: In an aspect, $R_1$ and $R_4$ are non-polar or low-polar ends, and are far away from a side of an electrode 10. In addition, in this application, contents of the first organic solvent and the second organic solvent are high. Therefore, a barrier can be formed, to effectively isolate other solvents in the electrolytic solution from the electrode 10 to avoid direct contact, reduce side reactions, and improve coulombic efficiency and cycling performance of the battery. In another aspect, a sulfinamide end and/or a sulfonamide end linked to $R_2$ and $R_3$ can be complexed with metal ions (for example, lithium ions) and more easily close to the side of the electrode 10, so that the metal ions (for example, the lithium ions) easily reach a surface of the electrode 10, thereby reducing a probability of dendrite formation, reducing a short circuit risk, and improving battery safety performance. In addition, the substituted sulfinamide or substituted sulfonamide compound can be preferentially reduced on the surface of the negative electrode than other components in the electrolytic solution, so that a stable interface film containing a metal fluoride, a metal nitride, a sulfide, and other compounds is formed on the surface of the negative electrode, reducing side reactions between the electrolytic solution and the negative electrode material, thereby improving coulombic efficiency and cycling stability of the battery. Moreover, the substituted sulfinamide or substituted sulfonamide compound can further form a stable interface film containing a metal fluoride, a sulfide, and other compounds on a surface of a high-voltage positive electrode material, suppressing further oxidation and decomposition of the electrolytic solution, thereby improving high-voltage stability of the electrolytic solution.

A metal halide formed in the interface film varies based on different secondary battery systems. Specifically, the metal halide may be lithium fluoride, sodium fluoride, potassium fluoride, or the like. The metal nitride may be lithium nitride, sodium nitride, potassium nitride, or the like.

In an implementation of this application, a specific structure of the first organic solvent shown in the formula (I) may be:

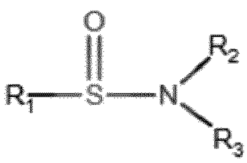 (1-1); or 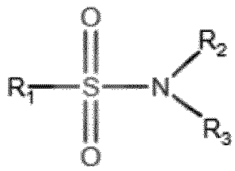 (1-2).

The compound shown in (1-1) is a substituted sulfinamide compound, and the compound shown in (1-2) is a substituted sulfonamide compound. In the compound (1-1) and the compound (1-2), $R_1$ is selected from one of fluoroalkyl, fluoroalkoxy, fluoroalkenyl, fluoroalkenyloxy, fluoroaryl, and fluoroaryloxy; and $R_2$ and $R_3$ are separately selected from one of alkyl, alkoxy, alkenyl, alkenyloxy, aryl, and aryloxy. $R_1$ is selected from the foregoing fluoro groups. The fluoro groups are non-polar or low-polar groups and are highly hydrophobic (incompatible with a polar solvent in the electrolytic solution). In the electrolytic solution, the $R_1$ end of the compound is far away from the electrode side, thereby preventing other components in the electrolytic solution from directly contacting the electrode and reducing side reactions. The sulfinamide or sulfonamide end linked to $R_2$ and $R_3$ can be complexed with metal ions (for example, lithium ions), and has high polarity, to be more easily close to the electrode side, so that the metal ions (for example, lithium ions) more easily reach the surface of the electrode, thereby reducing a probability of dendrite formation.

In an implementation of this application, a specific structure of the second organic solvent shown in the formula (II) may be:

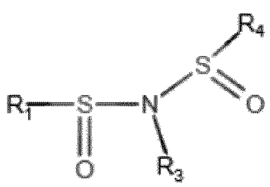 (2-1); 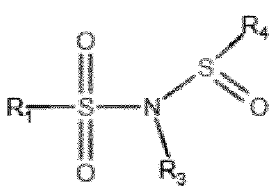 (2-2); or 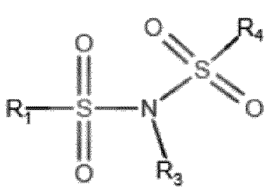 (2-3).

The compound shown in (2-1) is a substituted sulfinamide compound, the compound shown in (2-2) is a substituted sulfonamide-sulfinamide compound, and the compound shown in (2-3) is a substituted sulfonamide com-

pound. $R_1$ and $R_4$ are separately selected from one of fluoroalkyl, fluoroalkoxy, fluoroalkenyl, fluoroalkenyloxy, fluoroaryl, and fluoroaryloxy. $R_2$ and $R_3$ are separately selected from one of alkyl, alkoxy, alkenyl, alkenyloxy, aryl, and aryloxy. $R_1$ and $R_4$ are selected from the foregoing fluoro groups. The fluoro groups are non-polar or low-polar groups and are highly hydrophobic (incompatible with a polar solvent in the electrolytic solution). In the electrolytic solution, the $R_1$ and $R_4$ ends of the compound are far away from the electrode side, thereby preventing other components in the electrolytic solution from directly contacting the electrode and reducing side reactions. The sulfinamide end and/or the sulfonamide end linked to $R_3$ can be complexed with metal ions (for example, lithium ions), and has high polarity, to be more easily close to the electrode side, so that the metal ions (for example, lithium ions) more easily reach the surface of the electrode, thereby reducing a probability of dendrite formation.

In this implementation of this application, when the first organic solvent and the second organic solvent include a sulfinamide structure, the sulfinamide structure is more likely to be decomposed on the surface of the high-voltage positive electrode material than a sulfonamide structure, to form a stable interface film containing lithium fluoride, a sulfide, and other compounds, suppressing further oxidation and decomposition of the electrolytic solution, thereby improving high-temperature preservation stability of the electrolytic solution under a high voltage.

In an implementation of this application, the fluoroalkyl, fluoroalkoxy, fluoroalkenyl, fluoroalkenyloxy, fluoroaryl, or fluoroaryloxy may be partially or fully fluorinated. Full fluorination can provide better electrolyte-phobicity and lower polarity, so that it is more conducive to preventing other polar solvents in the electrolytic solution from directly contacting the electrode, thereby reducing side reactions.

In an implementation of this application, a number of carbon atoms in each of the fluoroalkyl, fluoroalkoxy, alkyl, and alkoxy may range from 1 to 20. Further, the number of carbon atoms may range from 1 to 10. Specifically, the number of carbon atoms is, for example, 1, 2, 3, 4, 5, 6, 7, 8, 9, or 10. For example, the fluoroalkyl may be specifically perfluoromethyl, perfluoroethyl, perfluoropropyl, or perfluorobutyl.

In an implementation of this application, a number of carbon atoms in each of the fluoroalkenyl, fluoroalkenyloxy, alkenyl, and alkenyloxy ranges from 2 to 10. Further, the number of carbon atoms may range from 2 to 10. Specifically, the number of carbon atoms is, for example, 2, 3, 4, 5, 6, 7, 8, 9, or 10. A number of carbon atoms in each of the fluoroaryl, fluoroaryloxy, aryl, or aryloxy ranges from 6 to 20. Further, the number of carbon atoms may range from 7 to 10. Specifically, the number of carbon atoms is, for example, 7, 8, 9, or 10.

In this implementation of this application, carbon chains of $R_1$ and $R_4$ are relatively long, which helps electrolyte-phobicity to better isolate other solvents in the electrolytic solution from the electrode to avoid direct contact. However, an excessively long carbon chain causes poor compatibility with the electrolytic solution. In some implementations, a number of carbon atoms in each of $R_1$ and $R_4$ may range from 2 to 6.

In this implementation of this application, carbon chains of $R_2$ and $R_3$ are relatively short, which can reduce steric hindrance, and helps metal ions to be close to O and N in molecular structures of the first organic solvent and the second organic solvent, for better complexing the metal ions. In some implementations, a number of carbon atoms in each of $R_2$ and $R_3$ may range from 1 to 3.

In an implementation of this application, the fluoroalkyl, fluoroalkoxy, fluoroalkenyl, fluoroalkenyloxy, fluoroaryl, fluoroaryloxy, alkyl, alkoxy, alkenyl, and alkenyloxy may be straight-chain or branched-chain.

In a specific implementation of this application, for example, molecular structural formulas of the first organic solvent and the second organic solvent may be shown in formulas (A) to (H).

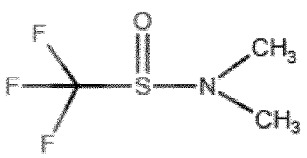

(A)

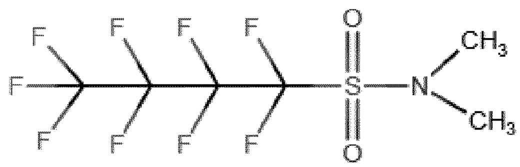

(B)

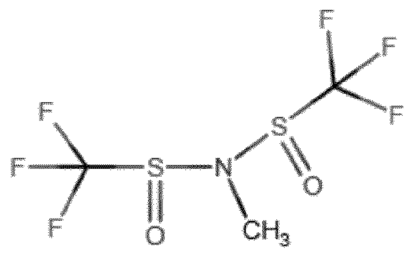

(C)

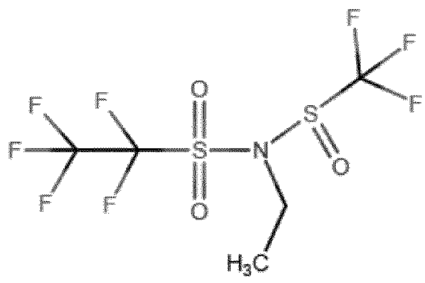

(D)

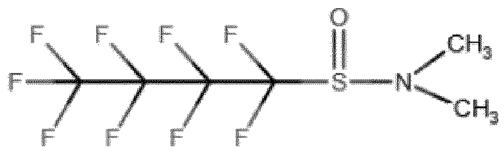

(E)

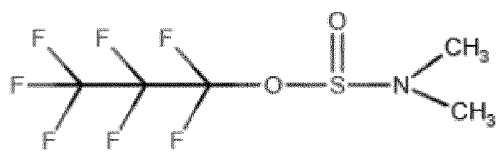

(F)

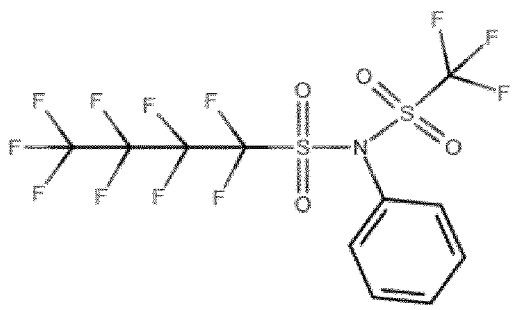

(G)

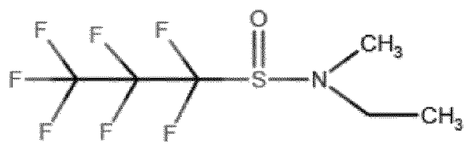

(H)

In an implementation of this application, to achieve better effect of reducing side reactions, a total content in mass of the first organic solvent and the second organic solvent in the electrolytic solution needs to be high, which specifically ranges from 10% to 90%. In some implementations of this application, the non-aqueous organic solvent may include only the first organic solvent. In some other implementations of this application, the non-aqueous organic solvent may include only the second organic solvent. In still some other implementations of this application, the non-aqueous organic solvent may include only the first organic solvent and the second organic solvent. If the content of the first organic solvent and/or the second organic solvent is high, there is still one part of the first organic solvent and the second organic solvent existing in the electrolytic solution while the other part is preferentially deposited on the surface of the negative electrode in an initial charging and discharging process, so as to block contact between the electrode and other components in the electrolytic solution, continuously reducing side reactions during charging and discharging of the battery. The first organic solvent and the second organic solvent are a substituted sulfinamide or substituted sulfonamide compound. If a total content in mass of the first organic solvent and the second organic solvent in the electrolytic solution is excessively high, corresponding conductivity decreases, causing a decrease of rate performance. To better improve comprehensive performance of the electrolytic solution, in some implementations of this application, a total content in mass of the first organic solvent and/or the second organic solvent in the electrolytic solution ranges from 20% to 50%. In some other implementations of this application, a total content in mass of the first organic solvent and/or the second organic solvent in the electrolytic solution ranges from 15% to 45%. In still some other implementations of this application, a total content in mass of the first organic solvent and/or the second organic solvent in the electrolytic solution ranges from 25% to 40%. In yet still some other implementations of this application, a total content in mass of the first organic solvent and/or the second organic solvent in the electrolytic solution ranges from 20% to 30%. Certainly, in some other implementations of this application, when the conductivity satisfies a requirement, a total content in mass of the first organic solvent and/or the second organic solvent in the electrolytic solution may range from 51% to 90%, specifically, for example, 51%, 55%, 60%, 70%, 80%, or 90%.

In an implementation of this application, the first organic solvent is a substituted sulfinamide or substituted

sulfonamide compound containing one polar end (a group end linked to $R_2$ and $R_3$) and one non-polar or low-polar end ($R_1$ end), and the second organic solvent is a substituted sulfinamide or substituted sulfonamide compound containing one polar end (a group end linked to $R_3$) and two non-polar or low-polar ends ($R_1$ and $R_4$ ends). A ratio of a non-polar or low-polar end (which is incompatible with a polar solvent in the electrolytic solution, and has electrolyte-phobicity) to a polar end (complexed with metal ions) can be adjusted by controlling a mass ratio of the first organic solvent to the second organic solvent in the electrolytic solution, so that synergistic effect of the first organic solvent and the second organic solvent is better achieved, making battery performance better.

In some implementations of this application, in the electrolytic solution, a mass ratio of the second organic solvent to the first organic solvent is controlled to range from 1:1 to 3:1. In some embodiments, a mass ratio of the second organic solvent to the first organic solvent may be specifically 1:1, 1.5:1, 2:1, 2.5:1, or 3:1. When the mass ratio of the second organic solvent to the first organic solvent is controlled to range from 1:1 to 3:1, polar end groups and non-polar end groups may be controlled to be in a more appropriate range. When the ratio of the second organic solvent to the first organic solvent is less than 1:1, there are a few non-polar end groups, which cannot fully block direct contact between the electrode and other components in the electrolytic solution. When the ratio of the second organic solvent to the first organic solvent is greater than 3: 1, there are a few polar end groups, making it weak to be complexed with metal ions, so that the capability of being close to the electrode side becomes weak.

In an implementation of this application, the first organic solvent and the second organic solvent may be used together with another solvent. To be specific, the non-aqueous organic solvent further includes a co-solvent, and the co-solvent includes one or more of a carbonate ester-based solvent, a carboxylate ester-based solvent, and an ether-based solvent.

In an implementation of this application, a ratio of a mass of the co-solvent to a total mass of the first organic solvent and/or the second organic solvent may range from 1:80 to 8:1, and specifically, may be comprehensively adjusted based on a specific battery system. In some implementations, a ratio of a mass of the co-solvent to a total mass of the first organic solvent and/or the second organic solvent may range from 1:50 to 4:1. In some implementations, a ratio of a mass of the co-solvent to a total mass of the first organic solvent and/or the second organic solvent may range from 1:20 to 1:1.

In an implementation of this application, the carbonate ester-based solvent includes cyclic carbonate or chain carbonate. The cyclic carbonate may be specifically but is not limited to one or more of ethylene carbonate (EC), propylene carbonate (PC), gamma-butyrolactone (GBL), and butylene carbonate (BC). The chain carbonate may be specifically but is not limited to one or more of dimethyl carbonate (DMC), ethyl methyl carbonate (EMC), diethyl carbonate (DEC), and dipropyl carbonate (DPC). The ether-based solvent includes cyclic ether or chain ether. The cyclic ether may be specifically but is not limited to one or more of 1,3-dioxolane (DOL), 1,4-dioxane (DX), crown ether, tetrahydrofuran (THF), 2-methyl tetrahydrofuran (2-$CH_3$-THF), and 2-trifluoromethyl tetrahydrofuran (2-$CF_3$-THF). The chain ether may be specifically but is not limited to one or more of dimethoxymethane (DMM), 1,2-dimethoxyethane (DME), and tetraethylene glycol dimethyl ether (TEGDME). The carboxylate ester-based solvent may be specifically but is not limited to one or more of methyl acetate (MA), ethyl acetate (EA), propyl acetate (EP), butyl acetate, propyl propionate (PP), and butyl propionate.

In an implementation of this application, an additive may be further added into the electrolytic solution based on different performance requirements. The additive may be specifically but is not limited to one or more of biphenyl, fluorobenzene, vinylene carbonate, trifluoromethyl ethylene carbonate, vinyl ethylene carbonate, 1,3-propane sultone, 1,4-butane sultone, ethylene sulfate, ethylene sulfite, methylene methanedisulfonate, butanedinitrile, hexanedinitrile, 1,2-bis(2-cyanoethoxy)ethane, and 1,3,6-hexanetricarbonitrile.

In an implementation of this application, based on different secondary battery systems, the electrolyte salt may include at least one of a lithium salt, a sodium salt, a potassium salt, a magnesium salt, a zinc salt, and an aluminum salt. Specifically, the lithium salt, the sodium salt, and the potassium salt may be one or more of $MClO_4$, $MBF_4$, $MPF_6$, $MAsF_6$, $MPF_2O_2$, $MCF_3SO_3$, MTDI, $MB(C_2O_4)_2$ (MBOB), $MBF_2C_2O_4$ (MDFOB), $M[(CF_3SO_2)_2N]$, $M[(FSO_2)_2N]$, and $M[(C_mF_{2m+1}SO_2) (C_nF_{2n+1}SO_2) N]$, where M is Li, Na, or K, and m and n are natural numbers. Similarly, the magnesium salt, the zinc salt, and the aluminum salt may be salt substances composed of magnesium ions, zinc ions, and aluminum ions with anions in the lithium salt, the sodium salt, and the potassium salt.

In an implementation of this application, a molar concentration of the electrolyte salt in the electrolytic solution ranges from 0.1 mol/L to 8.0 mol/L. Further, the molar concentration may range from 0.2 mol/L to 6 mol/L, from 0.5 mol/L to 4.0 mol/L, or from 0.8 mol/L to 2.0 mol/L.

Correspondingly, an embodiment of this application further provides a method for preparing the battery electrolytic solution. The method includes the following steps:

In an inert environment or a sealed environment (for example, a glove box filled with argon gas), the first organic solvent and/or the second organic solvent are/is mixed with a co-solvent to obtain a non-aqueous organic solvent. An additive is added into the non-aqueous organic solvent, then a fully dried electrolyte salt is dissolved in the non-aqueous organic solvent, and then the non-aqueous organic solvent with the dissolved electrolyte salt is stirred and uniformly

mixed, to obtain a battery electrolytic solution.

The operations in the foregoing preparation method may be implemented based on an existing conventional preparation process of an electrolytic solution. Specific selection of raw materials such as the electrolyte salt, the first organic solvent, the second organic solvent, the co-solvent, and the additive is described above, and details are not described herein again. It may be understood that, when the battery electrolytic solution does not include the additive, the electrolyte salt may be directly added into the non-aqueous organic solvent, and the non-aqueous organic solvent with the dissolved electrolyte salt is stirred and uniformly mixed, to obtain a battery electrolytic solution.

An embodiment of this application further provides a secondary battery, including a positive electrode, a negative electrode, a separator, and an electrolytic solution. The electrolytic solution is the battery electrolytic solution provided in embodiments of this application. The secondary battery provided in this embodiment of this application uses the battery electrolytic solution described above in this application, so that high coulombic efficiency and good cycling stability can be obtained. In an implementation of this application, the secondary battery may be a lithium secondary battery, a potassium secondary battery, a sodium secondary battery, a magnesium secondary battery, a zinc secondary battery, an aluminum secondary battery, or the like. The secondary battery provided in this embodiment of this application may be used for a terminal consumer product, such as a mobile phone, a tablet computer, a mobile power supply, a portable computer, a notebook computer, a digital camera, and another wearable or movable electronic device, an uncrewed aerial vehicle, an automobile, and the like, to improve product performance.

In an implementation of this application, the negative electrode may include one or more of a carbon-based negative electrode, a silicon-based negative electrode, a tin-based negative electrode, a lithium negative electrode, a sodium negative electrode, a potassium negative electrode, a magnesium negative electrode, a zinc negative electrode, and an aluminum negative electrode. The carbon-based negative electrode may include graphite, hard carbon, soft carbon, graphene, or the like. The silicon-based negative electrode may include silicon, silicon carbon, silicon oxygen, a silicon metal compound, or the like. The tin-based negative electrode may include tin, tin carbon, tin oxygen, or a tin metal compound. The lithium negative electrode may include a lithium metal or a lithium alloy. The lithium alloy may be specifically at least one of a lithium silicon alloy, a lithium sodium alloy, a lithium potassium alloy, a lithium aluminum alloy, a lithium tin alloy, and a lithium indium alloy. In this embodiment of this application, the electrolytic solution has good compatibility with a metal negative electrode, and also has oxidation resistance, thereby satisfying high-voltage characteristics.

In an implementation of this application, the positive electrode includes a positive electrode active material into which metal ions (lithium ions, sodium ions, potassium ions, magnesium ions, zinc ions, aluminum ions, or the like) can be reversibly intercalated/deintercalated. Selection of the positive electrode active material is not specially limited in this application, and may be a positive electrode active material that is commonly used for an existing secondary battery. A high-voltage positive electrode material may be used, to help prepare a high-voltage secondary battery. For example, a positive electrode active material of a lithium secondary battery may be lithium cobaltate ($LiCoO_2$), lithium iron phosphate ($LiFePO_4$), lithium-nickel-cobalt-manganese ($LiNi_{0.6}Co_{0.2}Mn_{0.2}$), or lithium polyanion compound $LiM_x(PO_4)_y$ (M is Ni, Co, Mn, Fe, Ti, or V, $0 \leq x \leq 5$, and $0 \leq y \leq 5$).

In an implementation of this application, a current collector of the positive electrode may be aluminum, titanium, tantalum, or an alloy thereof; and a current collector of the negative electrode may be copper, nickel, stainless steel, or the like.

In an implementation of this application, a separator may be an existing conventional separator, and may be a polymer separator, a non-woven fabric, or the like, including but not limited to a single-layer PP (polypropylene) separator, a single-layer PE (polyethylene) separator, a double-layer PP/PE separator, a double-layer PP/PP separator, and a three-layer PP/PE/PP separator.

A shape of the secondary battery in this embodiment of this application is not specifically limited, and may be a cylindrical shape, a button shape (coin shape), a flat shape, a square shape, or the like.

As shown in FIG. 3, an embodiment of this application further provides a terminal. The terminal 200 may be a mobile phone, or may be a product such as a tablet computer, a notebook computer, a portable computer, an intelligent wearable product, or an automobile. The terminal 200 includes a housing 201, and an electronic component and a battery (not shown in the figure) that are accommodated in the housing 201. The battery supplies power to the electronic component. The battery is the secondary battery provided in embodiments of this application. The housing 201 may include a front cover assembled at a front side of the terminal and a rear cover assembled at a rear side. The battery may be fixed at an inner side of the rear cover.

The following further describes embodiments of this application by using a plurality of examples.

Example 1

An electrolytic solution of a lithium secondary battery includes a lithium salt (lithium hexafluorophosphate $LiPF_6$), a non-aqueous organic solvent formed by mixing ethylene carbonate (EC) and ethyl methyl carbonate (EMC) in a mass

ratio of 50:50, a compound A: $CF_3$-S(=O)-N($CH_3$)$_2$, and ethylene sulfate (DTD). A concentration of the lithium salt ($LiPF_6$) is 1.0 mol/L. A percentage by mass of the compound A and a percentage by mass of ethylene sulfate (DTD) are 20% and 2% respectively.

Preparation of the electrolytic solution of the lithium secondary battery in this example is as follows:

In a glove box filled with argon gas, EC and EMC are mixed to form a non-aqueous organic solvent, the compound A and ethylene sulfate (DTD) are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery

In a percentage by mass, 2% of polyvinylidene fluoride (PVDF), 2% of conductive agent super P, and 96% of lithium cobaltate ($LiCoO_2$) are weighed and added into N-methylpyrrolidone (NMP) in sequence, and the N-methylpyrrolidone with the 2% polyvinylidene fluoride, 2% conductive agent super P, and 96% lithium cobaltate is fully stirred and uniformly mixed to obtain a slurry. The slurry is coated on an aluminum-foil current collector, and subject to drying, cold pressing, and slitting, to obtain a positive electrode plate.

In a percentage by mass, 1.5% of CMC, 2.5% of SBR, 1% of Super P, and 95% of graphite are weighed and added into deionized water in sequence, and the deionized water with 1.5% CMC, 2.5% SBR, 1% Super P, and 95% graphite is fully stirred and uniformly mixed to obtain a slurry. The slurry is coated on a copper-foil current collector, and subject to drying, cold pressing, and slitting, to obtain a negative electrode plate.

The positive electrode plate, the negative electrode plate, and a commercial PE separator are prepared into a cell. The cell is packaged with a polymer. The electrolytic solution of the lithium secondary battery prepared in Example 1 of this application is injected into the cell, and subject to formation and other processes, to obtain a soft-pack lithium secondary battery.

Example 2

An electrolytic solution of a lithium secondary battery includes a lithium salt ($LiPF_6$), a non-aqueous organic solvent formed by mixing ethylene carbonate (EC) and ethyl methyl carbonate (EMC) in a mass ratio of 50:50, a compound B: $CF_3CF_2CF_2CF_2$-S(=O)$_2$-N($CH_3$)$_2$, and ethylene sulfate (DTD). A concentration of the lithium salt ($LiPF_6$) is 1.0 mol/L. A percentage by mass of the compound B and a percentage by mass of ethylene sulfate (DTD) are 20% and 2% respectively.

Preparation of the electrolytic solution of the lithium secondary battery in this example is as follows:

In a glove box filled with argon gas, EC and EMC are mixed to form a non-aqueous organic solvent, the compound B and ethylene sulfate (DTD) are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 1.

Example 3

An electrolytic solution of a lithium secondary battery includes a lithium salt ($LiPF_6$), a non-aqueous organic solvent formed by mixing ethylene carbonate (EC), diethyl carbonate (DEC), and fluoroethylene carbonate (FEC) in a mass ratio of 30:60:10, and a compound C: $CF_3$-S(=O)-N($CH_3$)-S(=O)-$CF_3$. A concentration of the lithium salt ($LiPF_6$) is 1.0 mol/L. A percentage by mass of the compound C is 25%.

Preparation of the electrolytic solution of the lithium secondary battery in this example is as follows:

In a glove box filled with argon gas, EC, DEC, and FEC are mixed to form a non-aqueous organic solvent, the compound C is added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery

In a percentage by mass, 2% of polyvinylidene fluoride (PVDF), 2% of conductive agent super P, and 96% of lithium cobaltate ($LiCoO_2$) are weighed and added into N-methylpyrrolidone (NMP) in sequence, and the N-methylpyrrolidone with the 2% polyvinylidene fluoride, 2% conductive agent super P, and 96% lithium cobaltate is fully stirred and uniformly mixed to obtain a slurry. The slurry is coated on an aluminum-foil current collector, and subject to drying, cold pressing, and slitting, to obtain a positive electrode plate.

In a percentage by mass, 1.5% of CMC, 2.5% of SBR, 1% of Super P, and 95% of silicon carbon are weighed and added into deionized water in sequence, and the deionized water with the 1.5% CMC, 2.5% SBR, 1% Super P, and 95% silicon carbon is fully stirred and uniformly mixed to obtain a slurry. The slurry is coated on a copper-foil current collector, and subject to drying, cold pressing, and slitting, to obtain a negative electrode plate.
The positive electrode plate, the negative electrode plate, and a commercial PE separator are prepared into a cell. The cell is packaged with a polymer. The electrolytic solution of the lithium secondary battery prepared in Example 3 of this application is injected into the cell, and subject to formation and other processes, to obtain a soft-pack lithium secondary battery.

Example 4

An electrolytic solution of a lithium secondary battery includes a lithium salt ($LiPF_6$), a non-aqueous organic solvent formed by mixing ethylene carbonate (EC), diethyl carbonate (DEC), and fluoroethylene carbonate (FEC) in a mass ratio of 30:60:10, and a compound D: $CF_3CF_2$-S(=O)$_2$-N($CH_2CH_3$)-S(=O)-$CF_3$. A concentration of the lithium salt ($LiPF_6$) is 1.0 mol/L. A percentage by mass of the compound D is 20%.
Preparation of the electrolytic solution of the lithium secondary battery in this example is as follows:
In a glove box filled with argon gas, EC, DEC, and FEC are mixed to form a non-aqueous organic solvent, the compound D is added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.
Preparation of the lithium secondary battery is the same as that in Example 3.

Example 5

An electrolytic solution of a lithium secondary battery includes a lithium salt (lithium hexafluorophosphate $LiPF_6$ and lithium bis(fluorosulfonyl) imide LiFSI), a non-aqueous organic solvent formed by mixing dimethyl carbonate (DMC) and fluoroethylene carbonate (FEC) in a mass ratio of 50:50, a compound A: $CF_3$-S(=O)-N($CH_3$)$_2$, and vinylene carbonate (VC). A concentration of lithium hexafluorophosphate ($LiPF_6$) and a concentration of lithium bis(fluorosulfonyl) imide are 1.0 mol/L and 0.2 mol/L respectively. A percentage by mass of the compound A and a percentage by mass of vinylene carbonate (VC) are 15% and 2% respectively.
Preparation of the electrolytic solution of the lithium secondary battery in this example is as follows:
In a glove box filled with argon gas, DMC and FEC are mixed to form a non-aqueous organic solvent, the compound A and vinylene carbonate (VC) are added into the non-aqueous organic solvent, then the fully dried lithium salt ($LiPF_6$ and LiFSI) is dissolved in the solvent, and the solvent with the dissolved lithium salt is stirred and uniformly mixed, to obtain the electrolytic solution of the lithium secondary battery.

Preparation of the lithium secondary battery

In a percentage by mass, 2% of polyvinylidene fluoride (PVDF), 2% of conductive agent super P, and 96% of lithium cobaltate ($LiCoO_2$) are weighed and added into N-methylpyrrolidone (NMP) in sequence, and the N-methylpyrrolidone with the 2% polyvinylidene fluoride, 2% conductive agent super P, and 96% lithium cobaltate is fully stirred and uniformly mixed to obtain a slurry. The slurry is coated on an aluminum-foil current collector, and subject to drying, cold pressing, and slitting, to obtain a positive electrode plate.
The positive electrode plate, a lithium metal negative electrode plate, and a commercial PE separator are prepared into a cell. The cell is packaged with a polymer. The electrolytic solution of the lithium secondary battery prepared in Example 5 of this application is injected into the cell, and subject to formation and other processes, to obtain a soft-pack lithium secondary battery.

Example 6

An electrolytic solution of a lithium secondary battery includes a lithium salt (lithium hexafluorophosphate $LiPF_6$ and lithium bis(fluorosulfonyl) imide LiFSI), a non-aqueous organic solvent formed by mixing dimethyl carbonate (DMC) and fluoroethylene carbonate (FEC) in a mass ratio of 50:50, a compound E: $CF_3CF_2CF_2CF_2$-S(=O)-N($CH_3$)$_2$, and vinylene carbonate (VC). A concentration of lithium hexafluorophosphate ($LiPF_6$) and a concentration of lithium bis(fluorosulfonyl) imide (LiFSI) are 1.0 mol/L and 0.2 mol/L respectively. A percentage by mass of the compound E and a percentage by mass of vinylene carbonate (VC) are 15% and 2% respectively.
Preparation of the electrolytic solution of the lithium secondary battery in this example is as follows:
In a glove box filled with argon gas, DMC and FEC are mixed to form a non-aqueous organic solvent, the compound E

and vinylene carbonate (VC) are added into the non-aqueous organic solvent, then the fully dried lithium salt ($LiPF_6$ and LiFSI) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 7

An electrolytic solution of a lithium secondary battery includes a lithium salt (lithium hexafluorophosphate $LiPF_6$ and lithium bis(fluorosulfonyl) imide LiFSI), a non-aqueous organic solvent formed by mixing dimethyl carbonate (DMC) and fluoroethylene carbonate (FEC) in a mass ratio of 50:50, a compound B: $CF_3CF_2CF_2CF_2$-S(=O)$_2$-N(CH$_3$)$_2$, and vinylene carbonate (VC). A concentration of lithium hexafluorophosphate ($LiPF_6$) and a concentration of lithium bis(fluorosulfonyl) imide (LiFSI) are 1.0 mol/L and 0.2 mol/L respectively. A percentage by mass of the compound B and a percentage by mass of vinylene carbonate (VC) are 15% and 2% respectively.

Preparation of the electrolytic solution of the lithium secondary battery in this example is as follows:
In a glove box filled with argon gas, DMC and FEC are mixed to form a non-aqueous organic solvent, the compound B and vinylene carbonate (VC) are added into the non-aqueous organic solvent, then the fully dried lithium salt ($LiPF_6$ and LiFSI) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 8

An electrolytic solution of a lithium secondary battery includes a lithium salt (lithium bis(fluorosulfonyl) imide LiFSI and lithium difluoro(oxalato)borate LiDFOB), a non-aqueous organic solvent formed by mixing dimethyl carbonate (DMC), fluoroethylene carbonate (FEC), and 1,1,2,2-tetrafluoroethyl-2,2,3,3-tetrafluoropropylether (D2) in a mass ratio of 50:10:40, and a compound F: $CF_3CF_2CF_2O$-S(=O)-N(CH$_3$)$_2$. A concentration of LiFSI and a concentration of LiDFOB are 3.0 mol/L and 0.5 mol/L respectively. A percentage by mass of the compound F is 30%.

Preparation of the electrolytic solution of the lithium secondary battery in this example is as follows:
In a glove box filled with argon gas, DMC, FEC, and D2 are mixed to form a non-aqueous organic solvent, the compound F is added into the non-aqueous organic solvent, then the fully dried lithium salt (LiFSI and LiDFOB) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 9

An electrolytic solution of a lithium secondary battery includes a lithium salt (lithium bis(fluorosulfonyl) imide LiFSI and lithium difluoro(oxalato)borate LiDFOB), a non-aqueous organic solvent formed by mixing 1,2-dimethoxyethane (DME), fluoroethylene carbonate (FEC), and 1,1,2,2-tetrafluoroethyl-2,2,3,3-tetrafluoropropylether (D2) in a mass ratio of 50:10:40, and a compound G: $CF_3CF_2CF_2CF_2$-S(=O)$_2$-N(C$_6$H$_5$)-S(=O)$_2$-CF$_3$. A concentration of LiFSI and a concentration of LiDFOB are 5.0 mol/L and 0.5 mol/L respectively. A percentage by mass of the compound G is 20%.

Preparation of the electrolytic solution of the lithium secondary battery in this example is as follows:
In a glove box filled with argon gas, DME, FEC, and D2 are mixed to form a non-aqueous organic solvent, the compound G is added into the non-aqueous organic solvent, then the fully dried lithium salt (LiFSI and LiDFOB) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 10

An electrolytic solution of a lithium secondary battery includes a lithium salt (lithium bis(fluorosulfonyl) imide LiFSI and lithium difluoro(oxalato)borate LiDFOB), and a non-aqueous organic solvent composed of only a compound H: $CF_3CF_2CF_2$-S(=O)-N(CH$_3$)CH$_2$CH$_3$. A concentration of LiFSI and a concentration of LiDFOB are 2.0 mol/L and 0.5 mol/L respectively.

Preparation of the electrolytic solution of the lithium secondary battery in this example is as follows:
In a glove box filled with argon gas, the fully dried lithium salt (LiFSI and LiDFOB) is added into the non-aqueous organic solvent, the compound H, and the non-aqueous organic solvent with the dissolved dried lithium salt is stirred and uniformly mixed, to obtain the electrolytic solution of the lithium secondary battery.

Preparation of the lithium secondary battery is the same as that in Example 5.

Example 11

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, a compound A: $CF_3$-S(=O)-N($CH_3$)$_2$, and a compound C: $CF_3$-S(=O)-N($CH_3$)-S(=O)-$CF_3$. A concentration of lithium hexafluorophosphate ($LiPF_6$) is 1.0 mol/L. A percentage by mass of the compound A and a percentage by mass of the compound C are 10% and 10% respectively.

Preparation of the electrolytic solution of the lithium secondary battery in this example is as follows:
In a glove box filled with argon gas, DMC and FEC are mixed to form a non-aqueous organic solvent, the compound A and the compound C are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 12

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, a compound E: $CF_3CF_2CF_2CF_2$-S(=O)-N($CH_3$)$_2$, and a compound D: $CF_3$-S(=O)-N($CH_3CH_2$)-S(=O)$_2$-$CF_2CF_3$. A concentration of lithium hexafluorophosphate ($LiPF_6$) is 1.0 mol/L. A percentage by mass of the compound E and a percentage by mass of the compound D are 10% and 20% respectively.

Preparation of the electrolytic solution of the lithium secondary battery in this example is as follows:
In a glove box filled with argon gas, DMC and FEC are mixed to form a non-aqueous organic solvent, the compound E and the compound D are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 13

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, a compound B: $CF_3CF_2CF_2CF_2$-S(=O)$_2$-N($CH_3$)$_2$, and a compound D: $CF_3$-S(=O)-N($CH_3CH_2$)-S(=O)$_2$-$CF_2CF_3$. A concentration of lithium hexafluorophosphate ($LiPF_6$) is 1.0 mol/L. A percentage by mass of the compound B and a percentage by mass of the compound D are 10% and 15% respectively.

Preparation of the electrolytic solution of the lithium secondary battery in this example is as follows:
In a glove box filled with argon gas, DMC and FEC are mixed to form a non-aqueous organic solvent, the compound B and the compound D are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 14

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, a compound F: $CF_3CF_2CF_2$O-S(=O)-N($CH_3$)$_2$, and a compound G: $CF_3CF_2CF_2CF_2$-S(=O)$_2$-N($C_6H_5$)-S(=O)$_2$-$CF_3$. A concentration of lithium hexafluorophosphate ($LiPF_6$) is 1.0 mol/L. A percentage by mass of the compound F and a percentage by mass of the compound G are 10% and 30% respectively.

Preparation of the electrolytic solution of the lithium secondary battery in this example is as follows:
In a glove box filled with argon gas, DMC and FEC are mixed to form a non-aqueous organic solvent, the compound F and the compound G are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 15

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, a compound A: $CF_3$-S(=O)-N($CH_3$)$_2$, a compound B: $CF_3CF_2CF_2CF_2$-S(=O)$_2$-N($CH_3$)$_2$, and a compound C: $CF_3$-S(=O)-N($CH_3$)-S(=O)-$CF_3$. A concentration of lithium hexafluorophosphate ($LiPF_6$) is 1.0 mol/L. A percentage by mass of the compound A, a percentage by mass of the compound B, and a percentage by mass of the compound C are 10%, 10%, and 20% respectively.

Preparation of the electrolytic solution of the lithium secondary battery in this example is as follows:
In a glove box filled with argon gas, DMC and FEC are mixed to form a non-aqueous organic solvent, the compound A, the compound B, and the compound C are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 16

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, a compound A: $CF_3$-S(=O)-N($CH_3$)$_2$, and a compound C: $CF_3$-S(=O)-N($CH_3$)-S(=O)-$CF_3$. A concentration of lithium hexafluorophosphate ($LiPF_6$) is 1.0 mol/L. A percentage by mass of the compound A and a percentage by mass of the compound C are 10% and 2% respectively.

Preparation of the electrolytic solution of the lithium secondary battery in this example is as follows:
In a glove box filled with argon gas, DMC and FEC are mixed to form a non-aqueous organic solvent, the compound A and the compound C are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 17

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, a compound A: $CF_3$-S(=O)-N($CH_3$)$_2$, and a compound C: $CF_3$-S(=O)-N($CH_3$)-S(=O)-$CF_3$. A concentration of lithium hexafluorophosphate ($LiPF_6$) is 1.0 mol/L. A percentage by mass of the compound A and a percentage by mass of the compound C are 2% and 10% respectively.

Preparation of the electrolytic solution of the lithium secondary battery in this example is as follows:
In a glove box filled with argon gas, DMC and FEC are mixed to form a non-aqueous organic solvent, the compound A and the compound C are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 18

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, and a compound A: $CF_3$-S(=O)-N($CH_3$)$_2$. A concentration of lithium hexafluorophosphate ($LiPF_6$) is 1.0 mol/L. A percentage by mass of the compound A is 20%.

Preparation of the electrolytic solution of the lithium secondary battery in this example is as follows:
In a glove box filled with argon gas, DMC and FEC are mixed to form a non-aqueous organic solvent, the compound A is added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

Example 19

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, and a compound C: $CF_3$-S(=O)-N($CH_3$)-S(=O)-$CF_3$. A concentration of lithium hexafluorophosphate ($LiPF_6$) is 1.0 mol/L. A percentage by mass of the compound C is 20%.

Preparation of the electrolytic solution of the lithium secondary battery in this example is as follows:

In a glove box filled with argon gas, DMC and FEC are mixed to form a non-aqueous organic solvent, the compound C is added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 5.

**Comparative Example 1**

An electrolytic solution of a lithium secondary battery includes a lithium salt (lithium hexafluorophosphate $LiPF_6$), a non-aqueous organic solvent formed by mixing ethylene carbonate (EC) and ethyl methyl carbonate (EMC) in a mass ratio of 50:50, and ethylene sulfate (DTD). A concentration of the lithium salt ($LiPF_6$) is 1.0 mol/L. A percentage by mass of ethylene sulfate (DTD) is 2%.

Preparation of the electrolytic solution of the lithium secondary battery:

In a glove box filled with argon gas, EC and EMC are mixed to form a non-aqueous organic solvent, then ethylene sulfate (DTD) and the fully dried lithium salt ($LiPF_6$) are dissolved in the solvent, and the solvent with the dissolved lithium salt is stirred and uniformly mixed, to obtain the electrolytic solution of the lithium secondary battery.

Preparation of the lithium secondary battery is the same as that in Example 1.

**Comparative Example 2**

An electrolytic solution of a lithium secondary battery includes a lithium salt ($LiPF_6$), and a non-aqueous organic solvent formed by mixing ethylene carbonate (EC), diethyl carbonate (DEC), and fluoroethylene carbonate (FEC) in a mass ratio of 30:60:10. A concentration of the lithium salt ($LiPF_6$) is 1.0 mol/L.

Preparation of the electrolytic solution of the lithium secondary battery:

In a glove box filled with argon gas, EC, DEC, and FEC are mixed to form a non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.

Preparation of the lithium secondary battery is the same as that in Example 3.

**Comparative Example 3**

An electrolytic solution of a lithium secondary battery includes a lithium salt (lithium hexafluorophosphate $LiPF_6$ and lithium bis(fluorosulfonyl) imide LiFSI), a non-aqueous organic solvent formed by mixing dimethyl carbonate (DMC) and fluoroethylene carbonate (FEC) in a mass ratio of 50:50, N,N-dimethylacetamide ($CH_3$-C(=O)-N($CH_3$)$_2$) shown in a molecular structural formula (I), and vinylene carbonate (VC). A concentration of lithium hexafluorophosphate ($LiPF_6$) and a concentration of lithium bis(fluorosulfonyl) imide (LiFSI) are 1.0 mol/L and 0.2 mol/L respectively. A percentage by mass of N,N-dimethylacetamide and a percentage by mass of vinylene carbonate (VC) are 15% and 2% respectively.

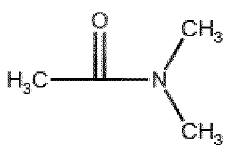

(I)

Preparation of the electrolytic solution of the lithium secondary battery:

In a glove box filled with argon gas, DMC and FEC are mixed to form a non-aqueous organic solvent, then N,N-dimethylacetamide, vinylene carbonate (VC), and the fully dried lithium salt ($LiPF_6$ and LiFSI) are dissolved in the solvent, and the solvent with the dissolved lithium salt is stirred and uniformly mixed, to obtain the electrolytic solution of the lithium secondary battery.

Preparation of the lithium secondary battery is the same as that in Example 5.

### Comparative Example 4

An electrolytic solution of a lithium secondary battery includes a lithium salt (lithium hexafluorophosphate $LiPF_6$ and lithium bis(fluorosulfonyl) imide LiFSI), a non-aqueous organic solvent formed by mixing dimethyl carbonate (DMC) and fluoroethylene carbonate (FEC) in a mass ratio of 50:50, N,N-dimethylperfluorobutanamide ($CF_3CF_2CF_2$-C(=O)-N$(CH_3)_2$) shown in a molecular structural formula (J), and vinylene carbonate (VC). A concentration of lithium hexafluorophosphate ($LiPF_6$) and a concentration of lithium bis(fluorosulfonyl) imide (LiFSI) are 1.0 mol/L and 0.2 mol/L respectively. A percentage by mass of N,N-dimethylperfluorobutanamide and a percentage by mass of vinylene carbonate (VC) are 15% and 2% respectively.

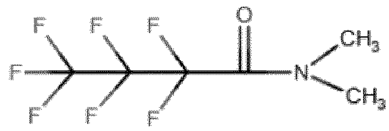 (J)

Preparation of the electrolytic solution of the lithium secondary battery:

In a glove box filled with argon gas, DMC and FEC are mixed to form a non-aqueous organic solvent, then N,N-dimethylperfluorobutanamide, vinylene carbonate (VC), and the fully dried lithium salt ($LiPF_6$ and LiFSI) are dissolved in the solvent, and the solvent with the dissolved lithium salt is stirred and uniformly mixed, to obtain the electrolytic solution of the lithium secondary battery.

Preparation of the lithium secondary battery is the same as that in Example 5.

### Comparative Example 5

An electrolytic solution of a lithium secondary battery includes a lithium salt (lithium hexafluorophosphate $LiPF_6$ and lithium bis(fluorosulfonyl) imide LiFSI), a non-aqueous organic solvent formed by mixing dimethyl carbonate (DMC) and fluoroethylene carbonate (FEC) in a mass ratio of 50:50, N,N-dimethylbutylsulfinamide ($CH_3CH_2CH_2CH_2$-S(=O)-N$(CH_3)_2$) shown in a molecular structural formula (K), and vinylene carbonate (VC). A concentration of lithium hexafluorophosphate ($LiPF_6$) and a concentration of lithium bis(fluorosulfonyl) imide (LiFSI) are 1.0 mol/L and 0.2 mol/L respectively. A percentage by mass of N,N-dimethylbutylsulfinamide and a percentage by mass of vinylene carbonate (VC) are 15% and 2% respectively.

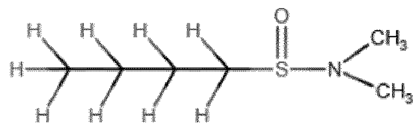 (K)

Preparation of the electrolytic solution of the lithium secondary battery:

In a glove box filled with argon gas, DMC and FEC are mixed to form a non-aqueous organic solvent, then N,N-dimethylbutylsulfinamide, vinylene carbonate (VC), and the fully dried lithium salt ($LiPF_6$ and LiFSI) are dissolved in the solvent, and the solvent with the dissolved lithium salt is stirred and uniformly mixed, to obtain the electrolytic solution of the lithium secondary battery.

Preparation of the lithium secondary battery is the same as that in Example 5.

### Comparative Example 6

An electrolytic solution of a lithium secondary battery includes a lithium salt (lithium bis(fluorosulfonyl) imide LiFSI and lithium difluoro(oxalato)borate LiDFOB), a non-aqueous organic solvent formed by mixing dimethyl carbonate (DMC), fluoroethylene carbonate (FEC), and 1,1,2,2-tetrafluoroethyl-2,2,3,3-tetrafluoropropylether (D2) in a mass ratio of 50:10:40, and a compound F: $CF_3CF_2CF_20$-S(=O)-N$(CH_3)_2$. A concentration of LiFSI and a concentration of LiDFOB are 3.0 mol/L and 0.5 mol/L respectively. A percentage by mass of the compound F is 3%. The difference between Comparative Example 6 and Example 8 only lies in different contents of the compound F.

Preparation of the electrolytic solution of the lithium secondary battery is the same as that in Example 8.

Preparation of the lithium secondary battery is the same as that in Example 5.

**Comparative Example 7**

An electrolytic solution of a lithium secondary battery includes a lithium salt (lithium bis(fluorosulfonyl) imide LiFSI and lithium difluoro(oxalato)borate LiDFOB), and a non-aqueous organic solvent formed by mixing 1,2-dimethoxyethane (DME), fluoroethylene carbonate (FEC), and 1, 1, 2,2-tetrafluoroethyl -2,2,3,3 -tetrafluoropropyl ether (D2) in a mass ratio of 50:10:40. A concentration of LiFSI and a concentration of LiDFOB are 5.0 mol/L and 0.5 mol/L respectively.
Preparation of the electrolytic solution of the lithium secondary battery:
In a glove box filled with argon gas, DME, FEC, and D2 are mixed to form a non-aqueous organic solvent, then the fully dried lithium salt (LiFSI and LiDFOB) is dissolved in the solvent, and the solvent with the dissolved lithium salt is stirred and uniformly mixed, to obtain the electrolytic solution of the lithium secondary battery.
Preparation of the lithium secondary battery is the same as that in Example 5.

**Comparative Example 8**

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, a compound A: $CF_3$-S(=O)-N($CH_3$)$_2$, and a compound C: $CF_3$-S(=O)-N($CH_3$)-S(=O)-$CF_3$. A concentration of lithium hexafluorophosphate ($LiPF_6$) is 1.0 mol/L. A percentage by mass of the compound A and a percentage by mass of the compound C are 4% and 1% respectively.
Preparation of the electrolytic solution of the lithium secondary battery in this example is as follows:
In a glove box filled with argon gas, DMC and FEC are mixed to form a non-aqueous organic solvent, the compound A and the compound C are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.
Preparation of the lithium secondary battery is the same as that in Example 5.

**Comparative Example 9**

An electrolytic solution of a lithium secondary battery includes a lithium salt (lithium hexafluorophosphate $LiPF_6$), a non-aqueous organic solvent formed by mixing dimethyl carbonate (DMC) and fluoroethylene carbonate (FEC) in a mass ratio of 70:30, a compound A: $CF_3$-S(=O)-N($CH_3$)$_2$, and a compound C: $CF_3$-S(=O)-N($CH_3$)-S(=O)-$CF_3$. A concentration of lithium hexafluorophosphate ($LiPF_6$) is 1.0 mol/L. A percentage by mass of the compound A and a percentage by mass of the compound C are 2% and 4% respectively.
Preparation of the electrolytic solution of the lithium secondary battery in this example is as follows:
In a glove box filled with argon gas, DMC and FEC are mixed to form a non-aqueous organic solvent, the compound A and the compound C are added into the non-aqueous organic solvent, then the fully dried lithium salt ($LiPF_6$) is dissolved in the solvent, and the solvent with the dissolved lithium salt is stirred and uniformly mixed, to obtain the electrolytic solution of the lithium secondary battery.
Preparation of the lithium secondary battery is the same as that in Example 5.
To provide strong support for beneficial effects brought by the technical solutions in Examples 1 to 19 and Comparative Examples 1 to 9, the following tests are provided:
Copper/lithium battery performance test: A copper positive electrode, a lithium metal negative electrode, and a separator are assembled into a button cell. 100 $\mu$L of electrolytic solution of the lithium secondary battery prepared in Examples 1 to 19 and Comparative Examples 1 to 9 is added dropwise. The test is carried out according to the following procedure. The test results are shown in Table 1.
The procedure for the copper/lithium battery test is set as follows: An initial charge/discharge current density is 0.5 mA/cm$^2$, an initial deposition amount is 4.0 mAh/cm$^2$, a cyclic discharge current density is 0.5 mA/cm$^2$, a cyclic charge current density is 1.5 mA/cm$^2$, a cyclic deposition amount is 1.0 mAh/cm$^2$, and a cycle number (n) is 50. Cyclic charge-discharge coulombic efficiency of the copper/lithium battery is calculated by comparing an initial discharge capacity ($Q_T$), a cyclic charge capacity (Qc), and a last charge capacity (Qs).
The coulombic efficiency (CE) of the battery is calculated according to the following formula:

$$CE_{avg} = \frac{nQ_C + Q_S}{nQ_C + Q_T} .$$

Lithium secondary battery performance test:

(1) Cycling performance test at room temperature: At room temperature (25±2°C), the lithium secondary battery obtained through assembling in Examples 1 to 19 and Comparative Examples 1 to 9 is charged at a constant current of 0.2 C to 4.45 V, and is then charged at a constant voltage until the charging current is less than or equal to 0.025 C. The battery is left to stand for 5 minutes between charging and discharging, and then discharged at a current of 0.5 C to a cutoff voltage of 3.0 V. A capacity retention rate after 100 cycles is recorded.

(2) 7-day preservation performance test at 60°C: At room temperature (25±2°C), the lithium secondary battery obtained through assembling in Examples 1 to 19 and Comparative Examples 1 to 9 is charged at a constant current of 0.2 C to 4.45 V, and is then charged at a constant voltage until the charging current is less than or equal to 0.025 C. The battery is left to stand for 5 minutes between charging and discharging, and then discharged at a constant current of 0.5 C to a cutoff voltage of 3.0 V. A capacity at this time is recorded as an initial capacity. The battery is fully charged in the preceding manner. Then, the battery is placed in a constant-temperature chamber at 60°C for seven days, and then taken out and placed at room temperature for two hours. After that, the battery is discharged at a constant current of 0.5 C to a cutoff voltage of 3.0 V. A capacity at this time is recorded as a remaining capacity.

(3) Low-temperature discharging performance test at -10°C: At room temperature (25±2°C), the lithium secondary battery obtained through assembling in Examples 1 to 19 and Comparative Examples 1 to 9 is charged at a constant current of 0.2 C to 4.45 V, and is then charged at a constant voltage until the charging current is less than or equal to 0.025 C. The battery is left to stand for 5 minutes between charging and discharging, and then discharged at a constant current of 0.5 C to a cutoff voltage of 3.0 V. A capacity at this time is recorded as an initial capacity. The battery is fully charged in the preceding manner. Then, the battery is placed in a constant-temperature chamber at -10°C for two hours. After that, the battery is discharged at a constant current of 0.5 C to a cutoff voltage of 3.0 V. A capacity at this time is recorded as a low-temperature discharging capacity.

The test results are shown in Table 1 and FIG. 4 to FIG. 8.

**Table 1 Test results of coulombic efficiency, cycling performance at room temperature, high-temperature preservation performance at 60°C, and low-temperature discharging performance at -10°C of batteries in Examples 1 to 19 and Comparative Examples 1 to 9**

| Example/ Comparative Example | Coulombic efficiency of copper/lithium battery/% | Capacity retention rate of cycling at room temperature/% | Remaining capacity retention rate of 7-day preservation at 60°C/ % | Capacity retention rate of low-temperature discharging at-10°C/% |
|---|---|---|---|---|
| Example 1 | 96.4 | 95.5 | 86.8 | 50.5 |
| Example 2 | 96.8 | 95.7 | 89.3 | 48.7 |
| Example 3 | 97.7 | 92.7 | 84.5 | 46.3 |
| Example 4 | 98.2 | 93.5 | 83.8 | 47.5 |
| Example 5 | 98.9 | 93.7 | 82.8 | 45.6 |
| Example 6 | 99.2 | 94.0 | 84.1 | 44.3 |
| Example 7 | 99.0 | 92.2 | 82.2 | 43.2 |
| Example 8 | 99.5 | 91.5 | 82.7 | 36.2 |
| Example 9 | 99.6 | 90.8 | 80.2 | 35.7 |
| Example 10 | 99.1 | 90.7 | 82.5 | 36.3 |
| Example 11 | 99.2 | 94.9 | 84.5 | 47.3 |
| Example 12 | 99.3 | 95.2 | 85.3 | 48.1 |
| Example 13 | 99.2 | 94.5 | 84.9 | 47.6 |
| Example 14 | 99.1 | 94.1 | 83.8 | 44.8 |
| Example 15 | 99.3 | 95.3 | 85.2 | 46.7 |
| Example 16 | 99.1 | 92.2 | 81.2 | 40.7 |
| Example 17 | 99.1 | 92.7 | 82.6 | 41.5 |
| Example 18 | 99.1 | 93.3 | 83.1 | 42.8 |

(continued)

| Example/ Comparative Example | Coulombic efficiency of copper/lithium battery/% | Capacity retention rate of cycling at room temperature/% | Remaining capacity retention rate of 7-day preservation at 60°C/ % | Capacity retention rate of low-temperature discharging at-10°C/% |
|---|---|---|---|---|
| Example 19 | 99.2 | 93.5 | 83.4 | 43.1 |
| Comparative Example 1 | 81.3 | 92.0 | 78.3 | 41.6 |
| Comparative Example 2 | 85.6 | 86.1 | 75.6 | 40.2 |
| Comparative Example 3 | 95.2 | 85.8 | 71.7 | 36.3 |
| Comparative Example 4 | 96.1 | 86.1 | 73.2 | 38.5 |
| Comparative Example 5 | 97.3 | 89.0 | 74.9 | 39.6 |
| Comparative Example 6 | 98.9 | 86.7 | 72.6 | 32.1 |
| Comparative Example 7 | 98.5 | 81.9 | 69.2 | 32.5 |
| Comparative Example 8 | 97.6 | 90.0 | 72.5 | 36.5 |
| Comparative Example 9 | 97.8 | 90.4 | 73.2 | 38.7 |

It can be learned from the test results in Table 1 and FIG. 4 that average 50-cycle coulombic efficiencies of the copper/lithium batteries in Examples 1 and 2 of this application are both higher than an average 50-cycle coulombic efficiency of the copper/lithium battery in Comparative Example 1, and capacity retention rates of cycling at room temperature for 100 cycles and low-temperature discharging performance of the lithium cobaltate/graphite batteries in Examples 1 and 2 of this application are both higher than a capacity retention rate of cycling at room temperature for 100 cycles and low-temperature discharging performance of the lithium cobaltate/graphite battery in Comparative Example 1. This indicates that performance of the battery can be significantly improved by using the electrolytic solution in this application. This is because the compound A and the compound B in the electrolytic solution can be preferentially reduced on the surfaces of the lithium metal negative electrode and the graphite negative electrode than other organic solvents, to form a stable interface film containing lithium fluoride, lithium nitride, a sulfide, and other components, which can not only reduce side reactions between the electrolytic solution and the lithium metal and graphite negative electrodes, but also improve coulombic efficiency, cycling performance, and low-temperature discharging performance of the battery. In addition, high-temperature preservation performance at 60°C of the lithium cobaltate/graphite batteries in Examples 1 and 2 of this application is higher than high-temperature preservation performance at 60°C of the lithium cobaltate/graphite battery in Comparative Example 1. This is because the compound A and the compound B can form, on the surface of the high-voltage positive electrode material, a stable interface film containing lithium fluoride, a sulfide, and other compounds, suppressing further oxidation and decomposition of the electrolytic solution, thereby improving high-temperature preservation stability of the electrolytic solution under a high voltage. Moreover, $CF_3$- and $CF_3CF_2CF_2CF_2$- in the compound A and the compound B are low-polar functional groups (which have electrolyte-phobicity, and are incompatible with other polar solvents in the electrolytic solution), and are far away from the graphite electrode side, which can isolate other solvents in the electrolytic solution from the graphite electrode to avoid direct contact, thereby reducing side reactions. In addition, the sulfinamide end and the sulfonamide end can be complexed with lithium ions, and more easily close to the graphite electrode side, so that the lithium ions easily reach the surface of the graphite electrode, thereby reducing a probability of lithium dendrite formation.

It can be learned from the test results in Table 1 and FIG. 5 that average 50-cycle coulombic efficiencies of the copper/lithium batteries in Examples 3 and 4 of this application are both higher than an average 50-cycle coulombic efficiency of the copper/lithium battery in Comparative Example 2, and capacity retention rates of cycling at room temperature for 100 cycles and low-temperature discharging performance of the lithium cobaltate/silicon carbon batteries in Examples 3 and 4 of this application are both higher than a capacity retention rate of cycling at room temperature for 100 cycles and low-temperature discharging performance of the lithium cobaltate/silicon carbon battery in Comparative Example 2. This indicates that the compound C and the compound D in the electrolytic solution can be preferentially reduced on the surfaces of the lithium metal negative electrode and the silicon carbon negative electrode than other organic solvents, to form a stable interface film containing lithium fluoride, lithium nitride, a sulfide, and other components, which can not only reduce side reactions between the electrolytic solution and the lithium metal and silicon carbon negative electrodes, but also improve coulombic efficiency, cycling performance, and low-temperature discharging performance of the battery. In addition, high-temperature preservation performance at 60°C of the lithium cobaltate/silicon carbon batteries in Examples 3 and 4 of this application is higher than high-temperature preservation performance at 60°C of the lithium cobaltate/silicon carbon battery in Comparative Example 2. This is because the compound C and the compound D can form, on the surface of the high-voltage positive electrode material, a stable interface film containing lithium fluoride, a sulfide, and other compounds, suppressing further oxidation and decomposition of the electrolytic solution, thereby improving high-temperature preservation stability of the electrolytic solution under a high voltage. Moreover, $CF_3$- and $CF_3CF_2$- in the compound C and the compound D are low-polar functional groups (which have electrolyte-phobicity, and are incompatible with other polar solvents in the electrolytic solution), and are far away from the silicon carbon electrode side, which can isolate other solvents in the electrolytic solution from the silicon carbon electrode to avoid direct contact, thereby reducing side reactions. In addition, the sulfinamide end and the sulfonamide end can be complexed with lithium ions, and more easily close to the silicon carbon electrode side, so that the lithium ions easily reach the surface of the silicon carbon electrode, thereby reducing a probability of lithium dendrite formation.

It can be learned from the test results in Table 1 and FIG. 6 that average 50-cycle coulombic efficiencies of the copper/lithium batteries in Examples 5 and 6 of this application are both higher than average 50-cycle coulombic efficiencies of the copper/lithium batteries in Comparative Examples 3 and 4, and capacity retention rates of cycling at room temperature for 100 cycles and low-temperature discharging performance of the lithium cobaltate/lithium batteries in Examples 5 and 6 of this application are both higher than capacity retention rates of cycling at room temperature for 100 cycles and low-temperature discharging performance of the lithium cobaltate/lithium batteries in Comparative Examples 3 and 4. This is because $CF_3$- and $CF_3CF_2CF_2CF_2$- in the compound A and the compound E are low-polar functional groups (which have electrolyte-phobicity, and are incompatible with other polar solvents in the electrolytic solution), and are far away from the lithium metal electrode side, which can isolate other solvents in the electrolytic solution from the lithium metal electrode to avoid direct contact, thereby reducing side reactions. In addition, the sulfinamide end can be complexed with lithium ions, and more easily close to the lithium metal electrode side, so that the lithium ions easily reach the surface of the lithium metal electrode, thereby reducing a probability of lithium dendrite formation. In another aspect, the compounds can be preferentially reduced on the surface of the lithium metal negative electrode to form a stable interface film containing lithium fluoride, lithium nitride, a sulfide, and other components, reducing side reactions between the electrolytic solution and the lithium metal negative electrode, thereby improving coulombic efficiency and cycling stability of the battery. In the compound I (N,N-dimethylacetamide) and the compound J (N,N-dimethylperfluorobutanamide) in the Comparative Examples, the amide bond and the lithium metal negative electrode form an unstable interface film containing lithium nitride, lithium alkyl, and other components, which causes serious side reactions, consequently leading to low coulombic efficiency and large impedance of the battery, and further leading to poor cycling performance at room temperature and low-temperature discharging performance of the battery. In addition, high-temperature preservation performance at 60°C of the lithium cobaltate/silicon lithium batteries in Examples 5 and 6 of this application is higher than high-temperature preservation performance at 60°C of the lithium cobaltate/lithium batteries in Comparative Examples 3 and 4. This is because the compound A and the compound E can form, on the surface of the high-voltage lithium cobaltate material, a stable interface film containing lithium fluoride, a sulfide, and other compounds, suppressing further oxidation and decomposition of the electrolytic solution, thereby improving high-temperature preservation stability of the electrolytic solution under a high voltage.

It can also be learned from the test results in Table 1 and FIG. 6 that an average 50-cycle coulombic efficiency of the copper/lithium battery in Example 6 of this application is higher than an average 50-cycle coulombic efficiency of the copper/lithium battery in Comparative Example 5, and a capacity retention rate of cycling at room temperature for 100 cycles of the lithium cobaltate/lithium battery in Example 6 of this application is higher than a capacity retention rate of cycling at room temperature for 100 cycles of the lithium cobaltate/lithium battery in Comparative Example 5. This is because $CF_3CF_2CF_2CF_2$- in the compound E is a low-polar functional group (which has electrolyte-phobicity, and is incompatible with other polar solvents in the electrolytic solution), and is far away from the lithium metal electrode side, which can isolate other solvents in the electrolytic solution from the lithium metal electrode to avoid direct contact, thereby reducing side reactions. In addition, the sulfinamide end can be complexed with lithium ions, and more easily close to the lithium metal electrode side, so that the lithium ions easily reach the surface of the lithium metal electrode, thereby reducing a probability of lithium dendrite formation. In the compound K (N,N-dimethylbutylsulfinamide) in the Comparative Example, the structure of the compound cannot function as an isolation barrier, and consequently other solvents in the

electrolytic solution are in direct contact with the lithium metal, causing serious side reactions.

It can also be learned from the test results in Table 1 and FIG. 6 that high-temperature preservation performance at 60°C for 100 cycles of the lithium cobaltate/lithium battery in Example 6 of this application is higher than high-temperature preservation performance at 60°C for 100 cycles of the lithium cobaltate/lithium battery in Example 7. This is because the sulfinamide structure in the compound E is more likely to be decomposed on the surface of the high-voltage lithium cobaltate material than the sulfonamide structure in the compound B, to form a stable interface film containing lithium fluoride, a sulfide, and other compounds, suppressing further oxidation and decomposition of the electrolytic solution, thereby improving high-temperature preservation stability of the electrolytic solution under a high voltage.

It can be learned from the test results in Table 1 and FIG. 7 that an average 50-cycle coulombic efficiency of the copper/lithium battery in Example 8 of this application is higher than an average 50-cycle coulombic efficiency of the copper/lithium battery in Comparative Example 6, and performance after 100 cycles of the lithium cobaltate/lithium battery in Example 8 of this application is higher than performance after 100 cycles of the lithium cobaltate/lithium battery in Comparative Example 6. By comparing the data in Example 8 with the data in Comparative Example 6 of this application, it can be learned that the substituted sulfinamide compound F, when used as an additive (≤ 5 wt%) in this application, does little to improve the battery performance. This is mainly because the compound F, as an additive, has a low content, and is not much left after battery formation and capacity grading, which is not enough to maintain cycling stability of the battery subsequently. However, in Example 8 of this application, the compound F is used as a solvent, so that it can be ensured that the interface film is continuously self-repaired after being damaged during battery cycling, thereby improving battery performance. In addition, if the compound F is added in an excessively small amount, a special structure (low-polar functional groups are far away from the lithium metal electrode side, and sulfinamide ends are close to the lithium metal electrode side) cannot be formed on the surface of the lithium metal electrode. This special structure can not only isolate other solvents in the electrolytic solution from the lithium metal electrode to avoid direct contact, but also reduce formation of lithium dendrites, and improve coulombic efficiency and high-temperature preservation performance.

It can also be learned from the test results in Table 1 and FIG. 7 that an average 50-cycle coulombic efficiency of the copper/lithium battery in Example 9 of this application is higher than an average 50-cycle coulombic efficiency of the copper/lithium battery in Comparative Example 7, and performance after 100 cycles of the lithium cobaltate/lithium battery in Example 9 of this application is higher than performance after 100 cycles of the lithium cobaltate/lithium battery in Comparative Example 7. This is because $CF_3$- and $CF_3CF_2CF_2CF_2$- in the compound G are low-polar functional groups (which have electrolyte-phobicity, and are incompatible with other polar solvents in the electrolytic solution), and are far away from the lithium metal electrode side, which can isolate other solvents in the electrolytic solution from the lithium metal electrode to avoid direct contact, thereby reducing side reactions. In addition, the sulfonamide end can be complexed with lithium ions, and more easily close to the lithium metal electrode side, so that the lithium ions easily reach the surface of the lithium metal electrode, thereby reducing a probability of lithium dendrite formation. In another aspect, the compounds can be preferentially reduced on the surface of the lithium metal negative electrode to form a stable interface film containing lithium fluoride, lithium nitride, a sulfide, and other components, reducing side reactions between the electrolytic solution and the lithium metal negative electrode, thereby improving coulombic efficiency and cycling stability of the battery. In addition, the compound can form, on the surface of the high-voltage lithium cobaltate material, a stable interface film containing lithium fluoride, a sulfide, and other compounds, suppressing further oxidation and decomposition of the electrolytic solution, thereby improving high-temperature preservation stability of the electrolytic solution under a high voltage. However, in Comparative Example 7, the substituted sulfonamide compound is not used, so that a stable interface film cannot be formed on the lithium metal negative electrode, which causes serious side reactions, consequently leading to low coulombic efficiency and poor performance of the battery.

By comparing the data in Examples 8 and 9 with the data in Comparative Examples 6 and 7 of this application, it can be learned that the substituted sulfonamide/substituted sulfinamide compound in this application is not only effective in the electrolytic solution containing a carbonate ester-based solvent, but also effective in the electrolytic solution containing an ether-based solvent.

It can also be learned from the test results in Table 1 and FIG. 7 that, when the compound H is used as the only solvent in Example 10 of this application, the copper/lithium battery still has good average 50-cycle coulombic efficiency, and the lithium cobaltate/lithium battery still has good performance.

It can also be learned from the test results in Table 1 and FIG. 8 that average 50-cycle coulombic efficiencies of the copper/lithium batteries in Examples 11 to 19 of this application are all higher than average 50-cycle coulombic efficiencies of the copper/lithium batteries in Comparative Examples 8 and 9, and capacity retention rates of cycling at room temperature for 100 cycles and low-temperature discharging performance of the lithium cobaltate/lithium batteries in Examples 11 to 19 of this application are all higher than capacity retention rates of cycling at room temperature for 100 cycles and low-temperature discharging performance of the lithium cobaltate/lithium batteries in Comparative Examples 8 and 9. This indicates that the first organic solvent and the second organic solvent can exert better synergistic effect in an appropriate ratio and an appropriate total content in mass, so that the battery performance is better. It can

be learned from FIG. 8 that, in Comparative Examples 8 and 9, when low contents (< 10%) of the first organic solvent and the second organic solvent limit improvement of the battery performance. This is mainly because the first organic solvent and the second organic solvent, as additives, have low contents, and are not much left after battery formation and capacity grading, which are not enough to maintain cycling stability of the battery subsequently. In addition, it can be learned by comparing Example 11 and Example 16 that, a mass ratio of the second organic solvent to the first organic solvent in Example 11 is equal to 1:1, a mass ratio of the second organic solvent to the first organic solvent in Example 16 is less than 1:1, and battery performance in Example 11 is better than battery performance in Example 16. This is mainly because non-polar ends of the compound added in the electrolytic solution are fewer, which are not enough to fully block direct contact between other components in the electrolytic solution and the lithium metal electrode, causing more side reactions. It can be learned by comparing Example 11 and Example 17 that, a mass ratio of the second organic solvent to the first organic solvent in Example 11 is equal to 1:1, a mass ratio of the second organic solvent to the first organic solvent in Example 17 is greater than 3: 1, and battery performance in Example 11 is better than battery performance in Example 17. This is mainly because polar ends of the compound added in the electrolytic solution are fewer, making it weak to be complexed with lithium ions, which is not conducive to the lithium ions being close to the lithium metal electrode side, consequently affecting uniform transport and deposition of the lithium ions.

It can also be learned from the test results in Table 1 that, by comparing Example 11 and Examples 18 and 19, the electrolytic solution in Example 11 includes both the first organic solvent and the second organic solvent in a mass ratio equal to 1:1, the electrolytic solution in Example 18 includes only the first organic solvent, the electrolytic solution in Example 19 includes only the second organic solvent, and battery performance in Example 11 is better than battery performance in Examples 18 and 19. This is mainly because by controlling a mass ratio of the first organic solvent to the second organic solvent in the electrolytic solution, a ratio of the non-polar or low-polar end (which is incompatible with the polar solvent in the electrolytic solution, and has electrolyte-phobicity) to the polar end (complexed with metal ions) can be adjusted, so that synergistic effect of the first organic solvent and the second organic solvent is better achieved, making battery performance better.

## Claims

1. A battery electrolytic solution, wherein the electrolytic solution comprises an electrolyte salt and a non-aqueous organic solvent, and the non-aqueous organic solvent comprises a first organic solvent shown in a general structural formula (I) and/or a second organic solvent shown in a general structural formula (II):

   $R_1\text{-}S(=O)_x\text{-}N(\text{-}R_3)\text{-}R_2$ formula (I);

   and

   $R_1\text{-}S(=O)_x\text{-}N(\text{-}R_3)\text{-}S(=O)y\text{-}R_4$ formula (II),

   wherein

   $R_1$ and $R_4$ are separately selected from one of fluoroalkyl, fluoroalkoxy, fluoroalkenyl, fluoroalkenyloxy, fluoroaryl, and fluoroaryloxy; $R_2$ and $R_3$ are separately selected from one of alkyl, alkoxy, alkenyl, alkenyloxy, aryl, and aryloxy; x is 1 or 2; and y is 1 or 2; and
   a total content in mass of the first organic solvent and/or the second organic solvent in the electrolytic solution ranges from 10% to 90%.

2. The battery electrolytic solution according to claim 1, wherein a number of carbon atoms in each of the fluoroalkyl, fluoroalkoxy, alkyl, and alkoxy ranges from 1 to 20; a number of carbon atoms in each of the fluoroalkenyl, fluoroalkenyloxy, alkenyl, and alkenyloxy ranges from 2 to 10; and a number of carbon atoms in each of the fluoroaryl, fluoroaryloxy, aryl, and aryloxy ranges from 6 to 20.

3. The battery electrolytic solution according to claim 1 or 2, wherein a total content in mass of the first organic solvent and/or the second organic solvent in the electrolytic solution ranges from 20% to 50%.

4. The battery electrolytic solution according to any one of claims 1 to 3, wherein the non-aqueous organic solvent further comprises a co-solvent, and the co-solvent comprises one or more of a carbonate ester-based solvent, a carboxylate ester-based solvent, and an ether-based solvent.

5. The battery electrolytic solution according to claim 4, wherein in the electrolytic solution, a ratio of a mass of the co-solvent to a total mass of the first organic solvent and/or the second organic solvent ranges from 1:80 to 8:1.

6. The battery electrolytic solution according to any one of claims 1 to 5, wherein the electrolytic solution further comprises an additive, and the additive comprises one or more of biphenyl, fluorobenzene, vinylene carbonate, trifluoromethyl ethylene carbonate, vinyl ethylene carbonate, 1,3-propane sultone, 1,4-butane sultone, ethylene sulfate, ethylene sulfite, methylene methanedisulfonate, butanedinitrile, hexanedinitrile, 1,2-bis(2-cyanoethoxy)ethane, and 1,3,6-hexanetricarbonitrile.

7. The battery electrolytic solution according to any one of claims 1 to 6, wherein the electrolyte salt comprises at least one of a lithium salt, a sodium salt, a potassium salt, a magnesium salt, a zinc salt, and an aluminum salt.

8. The battery electrolytic solution according to claim 7, wherein the electrolyte salt comprises one or more of $MClO_4$, $MBF_4$, $MPF_6$, $MAsF_6$, $MPF_2O_2$, $MCF_3SO_3$, MTDI, $MB(C_2O_4)_2$, $MBF_2C_2O_4$, $M[(CF_3SO_2)_2N]$, $M[(FSO_2)_2N]$, and $M[(C_mF_{2m+1}SO_2) (C_nF_{2n+1}SO_2) N]$, wherein M is Li, Na, or K, and m and n are natural numbers.

9. The battery electrolytic solution according to any one of claims 1 to 8, wherein a molar concentration of the electrolyte salt in the electrolytic solution ranges from 0.1 mol/L to 8.0 mol/L.

10. A secondary battery, comprising a positive electrode, a negative electrode, and a separator and an electrolytic solution between the positive electrode and the negative electrode, wherein the electrolytic solution comprises the battery electrolytic solution according to any one of claims 1 to 9.

11. The secondary battery according to claim 10, wherein the negative electrode comprises one or more of a carbon-based negative electrode, a silicon-based negative electrode, a tin-based negative electrode, a lithium negative electrode, a sodium negative electrode, a potassium negative electrode, a magnesium negative electrode, a zinc negative electrode, and an aluminum negative electrode.

12. The secondary battery according to claim 11, wherein the carbon-based negative electrode comprises one or more of graphite, hard carbon, soft carbon, and graphene; the silicon-based negative electrode comprises one or more of silicon, silicon carbon, silicon oxygen, and a silicon metal compound; the tin-based negative electrode comprises one or more of tin, tin carbon, tin oxygen, and a tin metal compound; and the lithium negative electrode comprises a lithium metal or a lithium alloy.

13. The secondary battery according to claim 12, wherein the lithium alloy comprises at least one of a lithium silicon alloy, a lithium sodium alloy, a lithium potassium alloy, a lithium aluminum alloy, a lithium tin alloy, and a lithium indium alloy.

14. The secondary battery according to claim 11, wherein the secondary battery comprises a lithium secondary battery, a potassium secondary battery, a sodium secondary battery, a magnesium secondary battery, a zinc secondary battery, or an aluminum secondary battery.

15. A terminal, comprising a housing, and an electronic component and a battery that are accommodated in the housing, wherein the battery supplies power to the electronic component, and the battery comprises the secondary battery according to any one of claims 10 to 14.

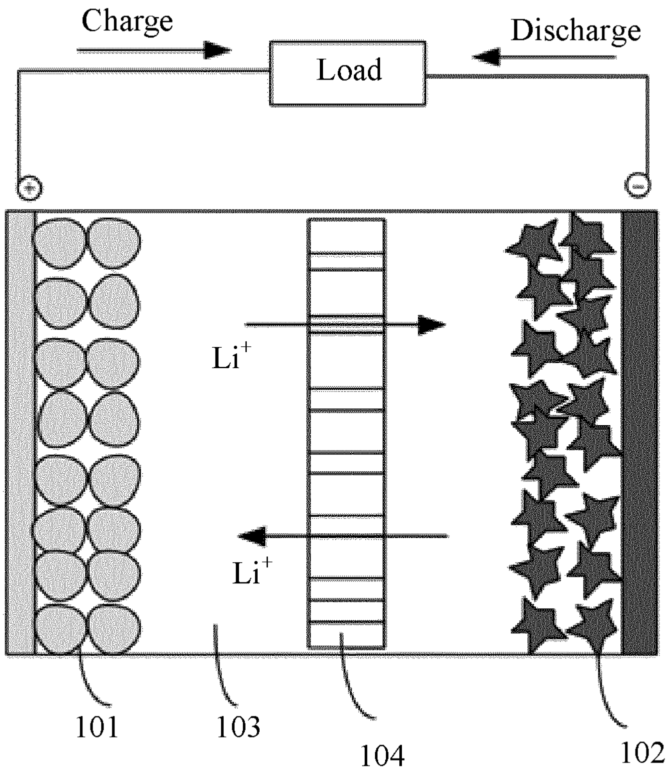


FIG. 1

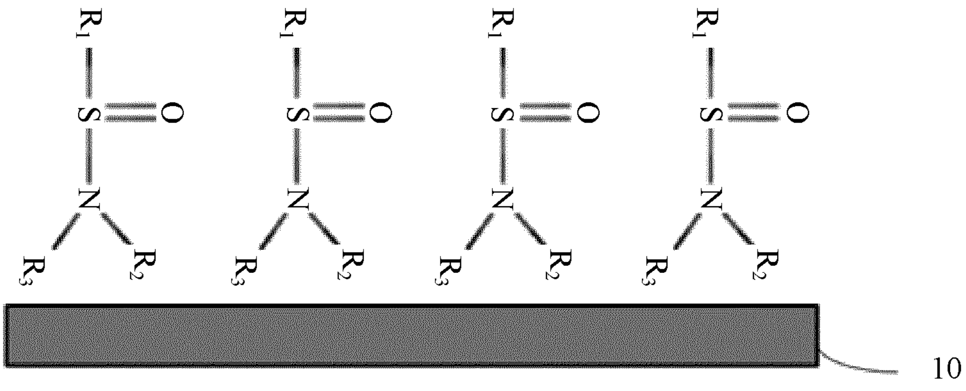


FIG. 2

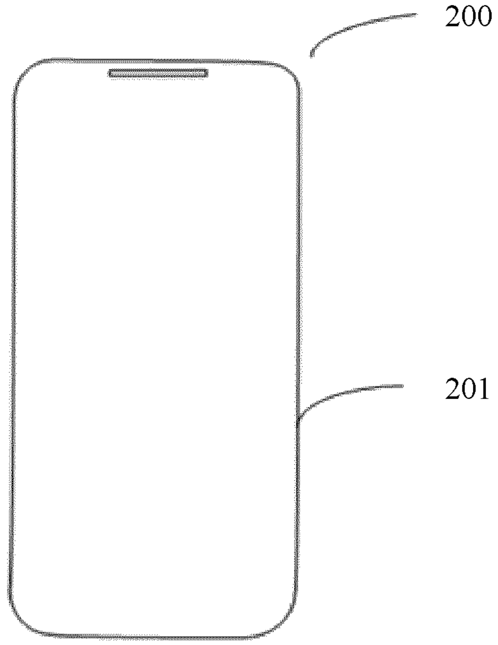
200
201

FIG. 3

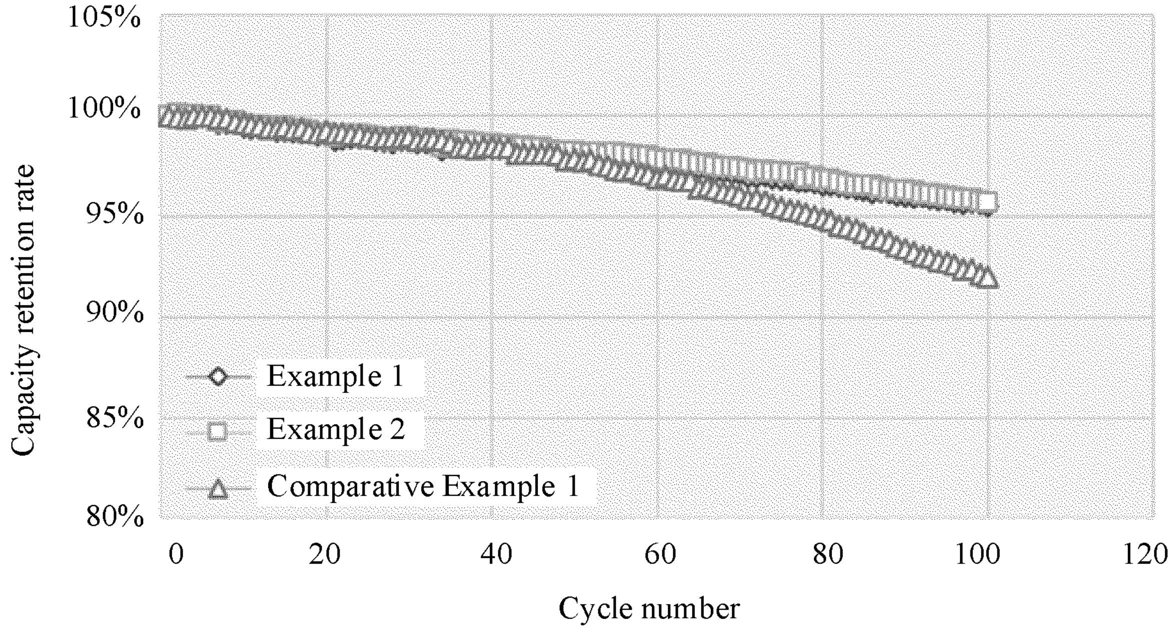
105%
100%
95%
90%
85%
80%
Capacity retention rate
0
20
40
60
80
100
120
Cycle number
Example 1
Example 2
Comparative Example 1

FIG. 4

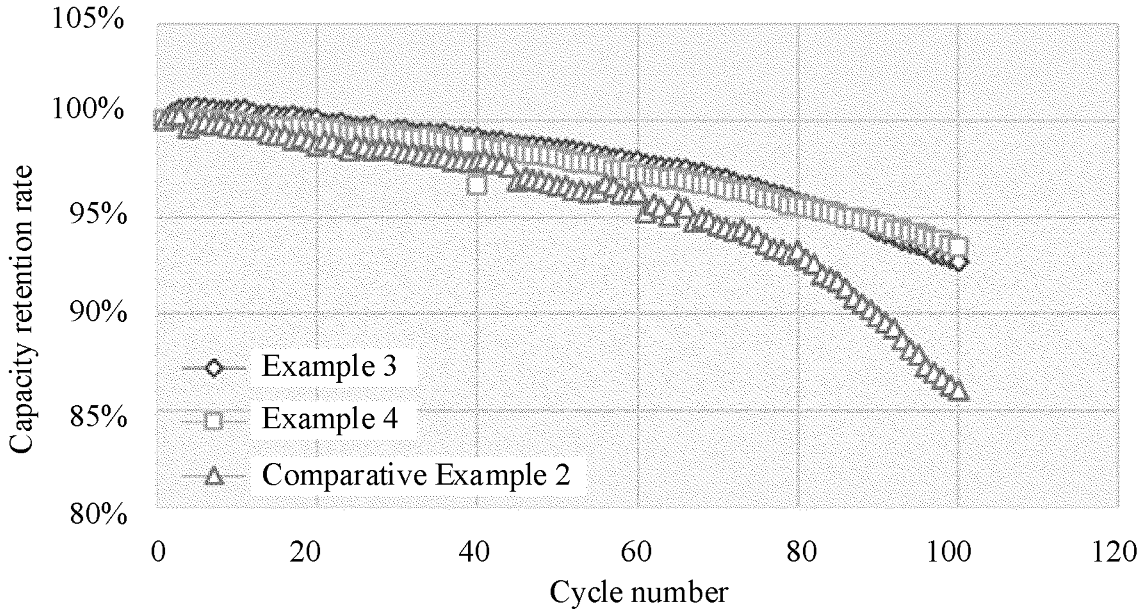
105%
100%
95%
90%
85%
80%
Capacity retention rate
0
20
40
60
80
100
120
Cycle number
Example 3
Example 4
Comparative Example 2

FIG. 5

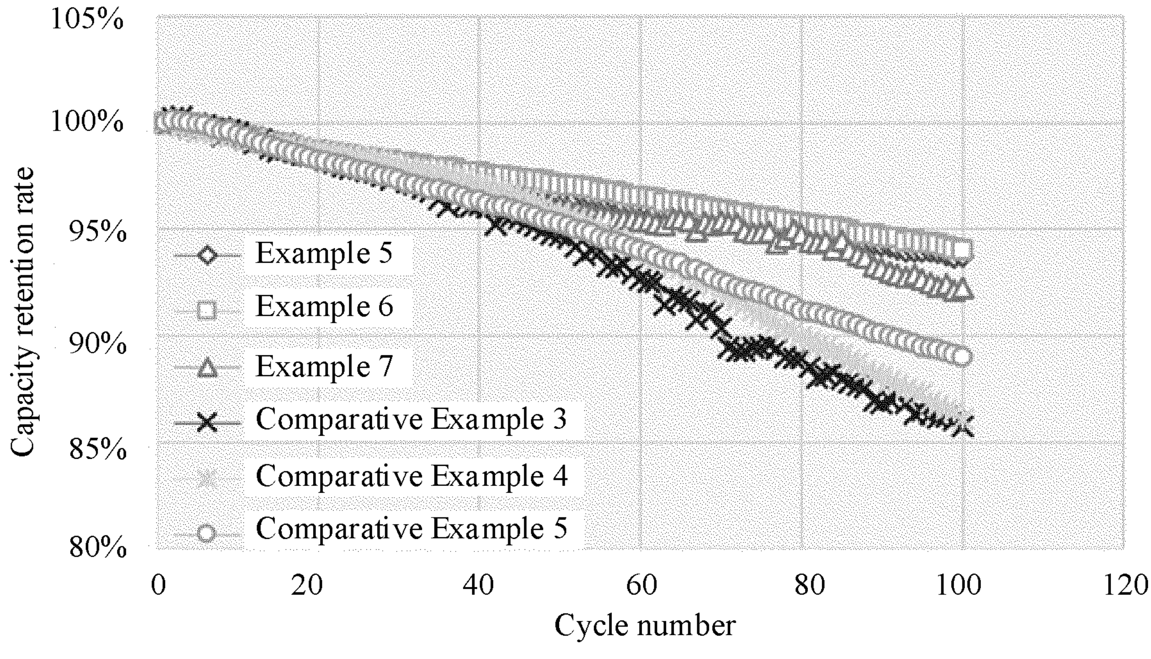
105%
100%
95%
90%
85%
80%
Capacity retention rate
0
20
40
60
80
100
120
Cycle number
Example 5
Example 6
Example 7
Comparative Example 3
Comparative Example 4
Comparative Example 5

FIG. 6

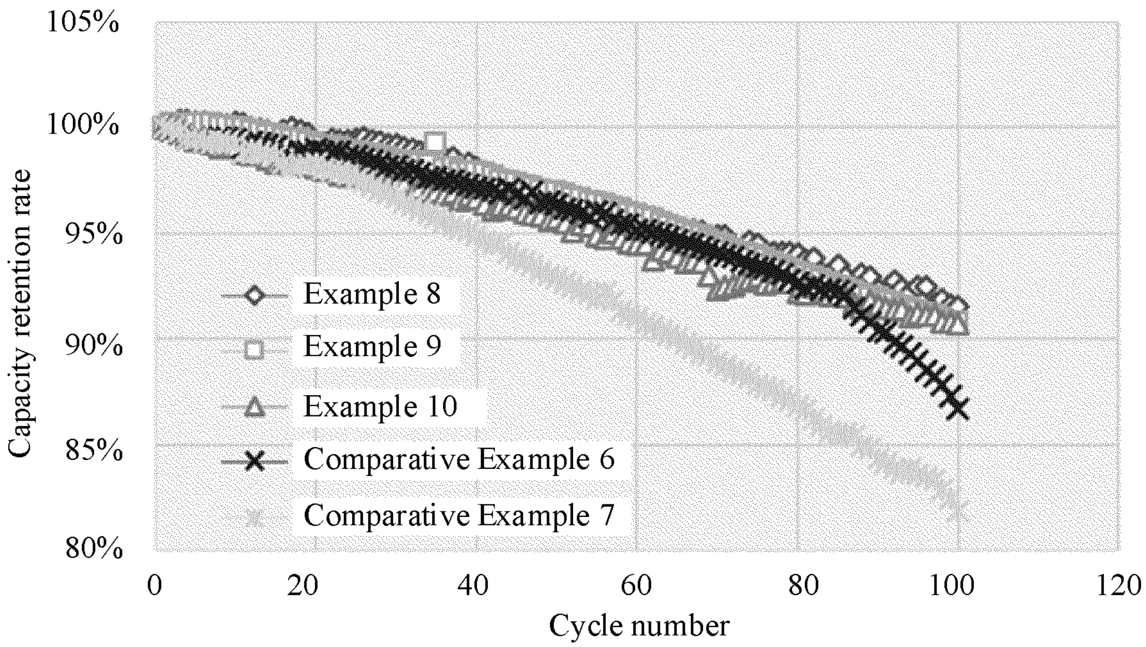

105%
100%
95%
90%
85%
80%
Capacity retention rate
Example 8
Example 9
Example 10
Comparative Example 6
Comparative Example 7
0
20
40
60
80
100
120
Cycle number


FIG. 7

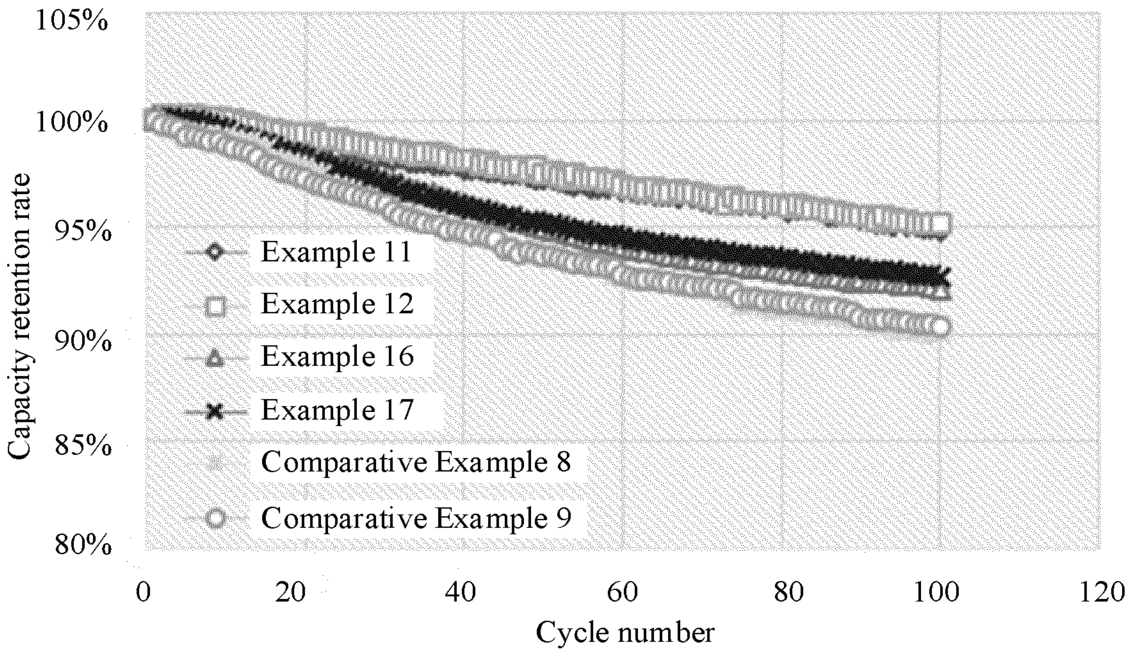

105%
100%
95%
90%
85%
80%
Capacity retention rate
Example 11
Example 12
Example 16
Example 17
Comparative Example 8
Comparative Example 9
0
20
40
60
80
100
120
Cycle number


FIG. 8

**INTERNATIONAL SEARCH REPORT**

International application No.
**PCT/CN2021/138148**

**A. CLASSIFICATION OF SUBJECT MATTER**

H01M 10/0569(2010.01)i；H01M 10/0567(2010.01)i

According to International Patent Classification (IPC) or to both national classification and IPC

**B. FIELDS SEARCHED**

Minimum documentation searched (classification system followed by classification symbols)

H01M

Documentation searched other than minimum documentation to the extent that such documents are included in the fields searched

Electronic data base consulted during the international search (name of data base and, where practicable, search terms used)

CNABS; CNTXT; WPABSC; VEN; ENTXT; ENTXTC; CNKI; STN: 电解液, 溶剂, 磺酰胺, 磺酰亚胺, 氟, 质量, 含量, electrolyte, solvent, sulfonamide, sulfonimide, fluorine, mass, content

**C. DOCUMENTS CONSIDERED TO BE RELEVANT**

| Category* | Citation of document, with indication, where appropriate, of the relevant passages | Relevant to claim No. |
|---|---|---|
| X | CN 1289765 A (MERCK PATENT GMBH) 04 April 2001 (2001-04-04)<br>description page 1 paragraph 1 - page 14 paragraph 7 | 1-15 |
| Y | CN 1289765 A (MERCK PATENT GMBH) 04 April 2001 (2001-04-04)<br>description page 1 paragraph 1 - page 14 paragraph 7 | 1-15 |
| Y | CN 101997139 A (SONY CORPORATION) 30 March 2011 (2011-03-30)<br>description paragraphs 4, 43-82, 112-122, 206, 501 | 1-15 |
| Y | CN 103515650 A (HUAZHONG UNIVERSITY OF SCIENCE AND TECHNOLOGY) 15 January 2014 (2014-01-15)<br>description, paragraphs 11-60 | 1-15 |
| X | EP 3050872 A1 (UNIV MUENSTER WESTFAELISCHE WILHELMS et al.) 03 August 2016 (2016-08-03)<br>description, paragraphs 2-66 | 1-15 |
| Y | EP 3050872 A1 (UNIV MUENSTER WESTFAELISCHE WILHELMS et al.) 03 August 2016 (2016-08-03)<br>description, paragraphs 2-66 | 1-15 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **14 February 2022** | **01 March 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No. **PCT/CN2021/138148**

**C. DOCUMENTS CONSIDERED TO BE RELEVANT**

| Category* | Citation of document, with indication, where appropriate, of the relevant passages | Relevant to claim No. |
|---|---|---|
| Y | CN 110176631 A (GUANGZHOU TINCI MATERIALS TECHNOLOGY CO., LTD.) 27 August 2019 (2019-08-27)<br>description paragraphs 31-137 | 1-15 |
| A | CN 109378523 A (LIYANG ZHONGKE HAINA TECHNOLOGY CO., LTD.) 22 February 2019 (2019-02-22)<br>entire document | 1-15 |

Form PCT/ISA/210 (second sheet) (January 2015)

**INTERNATIONAL SEARCH REPORT**
**Information on patent family members**

International application No.
**PCT/CN2021/138148**

| Patent document cited in search report | Publication date (day/month/year) | Patent family member(s) | Publication date (day/month/year) |
|---|---|---|---|
| CN 1289765 A | 04 April 2001 | EP 1088814 A1 | 04 April 2001 |
| | | KR 20010067251 A | 12 July 2001 |
| | | BR 0004449 A | 10 April 2001 |
| | | JP 2001097944 A | 10 April 2001 |
| | | CA 2321373 A1 | 29 March 2001 |
| | | DE 19953051 A1 | 05 April 2001 |
| | | DE 19953638 A1 | 10 May 2001 |
| CN 101997139 A | 30 March 2011 | JP 2011044352 A | 03 March 2011 |
| | | KR 20110020181 A | 02 March 2011 |
| | | US 2011045357 A1 | 24 February 2011 |
| CN 103515650 A | 15 January 2014 | None | |
| EP 3050872 A1 | 03 August 2016 | EP 3050872 B1 | 03 October 2018 |
| CN 110176631 A | 27 August 2019 | US 2021384554 A1 | 09 December 2021 |
| | | EP 3819976 A1 | 12 May 2021 |
| | | WO 2020248565 A1 | 17 December 2020 |
| CN 109378523 A | 22 February 2019 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202011490108